# EUROPEAN PATENT SPECIFICATION

(11) **EP 4 590 852 B1**
(45) Date of publication and mention of the grant of the patent: **22.04.2026**
(21) Application number: 23782145.9
(22) Date of filing: 22.09.2023
(51) Int. Cl.: C12Q 1/6832, C12Q 1/6841, C12Q 1/6853

(54) **METHOD OF DETECTION OF A TARGET NUCLEIC ACID SEQUENCE**
VERFAHREN ZUM NACHWEIS EINER ZIELNUKLEINSÄURESEQUENZ
PROCÉDÉ DE DÉTECTION D'UNE SÉQUENCE D'ACIDE NUCLÉIQUE CIBLE

(30) Priority: 23.09.2022 GB 202213959
(43) Date of publication of application: 30.07.2025
(73) Proprietor: Readily AB, 750 02 Uppsala (SE)
(72) Inventor: LANDEGREN, Ulf, 751 08 Uppsala (SE); XU, Bo, 753 18 Uppsala (SE); LÖF, Liza, 751 08 Uppsala (SE); KAMALI-MOGHADDAM, Masood, 751 08 Uppsala (SE)
(74) Representative: Barker Brettell Sweden AB
(86) International application number: PCT/EP2023/076299
(87) International publication number: WO 2024/062126

(56) References cited:
- CN-A- 112 410 401
- US-A1- 2021 238 680
- PEIDONG SHEN ET AL: "Multiplex target capture with double-stranded DNA probes", GENOME MEDICINE, vol. 5, no. 5, 1 January 2013 (2013-01-01), pages 50, XP055146836, ISSN: 1756-994X, DOI: 10.1186/gm454

## Description

### Field

The present disclosure and invention concerns the field of nucleic acid detection. Particularly, the present disclosure and invention relates to a method for detecting a nucleic acid target molecule using padlock probes and rolling circle amplification (RCA). The method herein provides a new way of removing free padlock probes which have failed to hybridise to their target, or to be ligated, and involves the use of an oligonucleotide, termed herein a silencing oligonucleotide, to "block" such unbound, or unligated, padlock probes. Also provided are silencing oligonucleotides and kits for use in such a method.

### Background

The detection of target nucleic acid molecules has applications in many different fields, including notably clinically in the diagnosis, prognosis and/or treatment of disease, as well as in research and biosecurity. Further nucleic acids are frequently used as tags or labels in reporter systems for detecting other analytes, where the nucleic acid is detected as a proxy, or indicator for the analyte in question.

Target nucleic acid sequences may readily be detected using labelled hybridisation probes, but simple hybridisation probes have relatively high lower detection limits, and cannot readily be used to discriminate between similar nucleic acid sequences. To increase sensitivity, target nucleic acid molecules containing target sequences may typically amplified, to increase the amount of target sequence available for detection. RCA is commonly used for such amplification, and is typically used in conjunction with padlock probes.

RCA utilises a strand displacement polymerase enzyme, and requires a circular amplification template, which may be provided by a circularised padlock probe. Amplification of the circular template provides a concatenated RCA product, comprising multiple copies of a sequence complementary to that of the amplification template. Such a concatemer typically forms a ball or "blob", which may readily be visualised and detected, and thus RCA-based assays have been adopted for the detection of nucleic acids, and indeed, more generally, as reporter systems for the detection of any target analyte.

Padlock probes are typically linear oligonucleotides with two separate target-complementary binding regions, connected by an intervening "backbone" region. When the probe has bound (hybridised) to its target nucleic acid sequence, the ends of the probe may be ligated together to circularise the probe. The circularised padlock probe may then be used as the template for a RCA reaction, and the RCA product may be detected. This forms the basis of a number of detection assays in use today. Padlock probes are highly specific, since they require dual recognition, or two binding sites for a target nucleic acid sequence. They further provide an extra layer of specificity, since only probes correctly base-paired at the ligation site will be ligated to generate the template for the molecule which will be detected. When the padlock probe hybridises to the target nucleic acid sequence with its target-binding regions directly adjacent to one another, the ends of the padlock probe may be ligated to each other directly. Alternatively, the target-binding regions of the padlock probe may hybridise to the target nucleic acid with a gap in between, and the gap may be filled in, either by hybridisation of one or more gap oligonucleotides in the gap region, or by a polymerase-catalysed extension of the hybridised 3 'end of the probe. In this way, the hybridised ends of the padlock probe may be ligated to each other indirectly, in that they are each hybridised to an intervening "gap sequence". Such "gap fill" padlock probes are also known as molecular inversion probes.

Generally speaking, in a RCA-based method using padlock probes it is desired to remove unbound padlock probes, that is padlock probes which have not hybridised to their target nucleic acid (i.e. free padlock probes) or which have not become ligated before the RCA reaction is performed. In this way, mis-priming by free padlock probes present in the reaction mixture can be avoided, for example by hybridising the product of the RCA reaction. More particularly, free padlock probes which subsequently hybridise to the RCA product (RCP) generated in the RCA reaction can prime extension reactions templated by the RCP, leading to double-stranded products, to which detection oligonucleotides for detection of the RCP cannot bind. By removing, or "neutralising", such free padlocks such that they cannot participate in subsequent hybridisation or priming reactions, specificity and/or sensitivity of the method may be improved.

Current measures for removing free padlocks include washing. However, this requires a solid phase and thus is not applicable to homogenous, or so-called "in solution" methods, in which a solid phase is not used. Free padlocks may also be removed by enzymatic digestion, most notably using exonuclease enzymes which are able to digest nucleic acids with free, or unligated ends, but sparing circularised padlock probes, which do not have a free end. However, the disadvantage of this is not only the expense of an additional enzyme, but also the need to inactivate the exonuclease, so as to prevent the digestion of other assay/reaction components down the line (including the RCP, for example). Such inactivation is generally performed by heating up to 95 °C. In order to be able to run the assay in one tube in solution, without extra enzymatic steps, heating steps or washing steps, another solution is required.

Shen et al., Multiplex target capture with double-stranded DNA probes, Genome Medicine 2013, 5: 50 discloses target capture using double-stranded probes, which consist of single-stranded, complementary long padlock probes, each selectively capturing one strand of a genomic target through circularization.

US 2021/238680 A1 discloses methods for increasing capture efficiency of a spatial array using rolling circle amplification of a padlock probe that hybridizes to a capture probe. Also provided are methods for using such spatial arrays to detect a biological analyte in a biological sample.

### Summary

The present inventors have devised an alternative solution to the problem of removing unwanted unhybridised padlock probes in a padlock probe and RCA-based assay method.

This involves the use of an oligonucleotide, termed herein a "silencing oligonucleotide", which is able to hybridise to the ends of the padlock probe, and prevent them from participating in further reactions. In effect, the padlock probe becomes "closed" and is functionally inactivated, unless a correct template is present. In other words, the padlock probe is rendered inert. To ensure that the ends of the padlock probe cannot become ligated to one another in a ligation reaction templated by the silencing oligonucleotide, the padlock probe-binding regions of the silencing oligonucleotide are separated by a gap, which keeps the ends of the padlock probe apart from one another when it is hybridised to the silencing oligonucleotide.

Accordingly, in a first aspect provided herein is a method for detecting a target nucleic acid molecule in a sample, said method comprising:
(i) contacting the sample with a padlock probe, which comprises at its 5' and 3 'ends target-binding regions which are complementary to probe-binding sites in the target nucleic acid molecule;
(ii) contacting the padlock probe with a silencing oligonucleotide which comprises first and second silencing regions which are complementary to the target-binding regions at the ends of the padlock probe, wherein the two silencing regions are separated from one another by at least 5 nucleotides, and wherein said contacting occurs before, during or after contacting the sample with the padlock probe;
(iii) allowing the target-specific binding regions of the padlock probe to hybridise to the target nucleic acid molecule in juxtaposition for ligation, directly or indirectly, to each other;
(iv) ligating, directly or indirectly, the 5' and 3 'ends of the padlock probe to circularise the padlock probe;
(v) after the silencing oligonucleotide has been contacted with the padlock probe, contacting the sample containing the circularised padlock probe with a strand-displacement polymerase and performing a RCA reaction using the circularised padlock probe as a RCA template to generate a RCA product (RCP);
(vi) during or after the RCA reaction, detecting the RCP to detect the target nucleic acid molecule.

The silencing oligonucleotide can be viewed as a blocking oligonucleotide for the padlock probe. Further, since the silencing oligonucleotide performs an equivalent function to removing unhybridised or unligated padlocks by washing it may also be termed a "washing oligonucleotide" (and by analogy the silencing regions of the oligonucleotide may alternatively be termed "washing regions").

A feature of the method is that the RCA reaction does not take place, i.e. is not initiated, until after the step of contacting the silencing oligonucleotide with the padlock probe. In other words, RCA starts after the silencing oligonucleotide is present in the sample or reaction mixture (and is available to bind to any free padlock probes). More particularly, the RCA reaction is performed (i.e. initiated) after the contacting with silencing oligonucleotide and after the ligation of target-hybridised padlock probes. More particularly, the RCA reaction is not initiated until after said contacting, or after said contacting and ligation, although this can be very shortly after.

A RCA reaction is typically initiated by adding one or more reagents for the RCA (termed "RCA reagents"). This will by typically be the polymerase enzyme for RCA and nucleotides (specifically dNTPs), although optionally a primer for RCA may also be added. One or more of the RCA reagents may be added earlier, as long as all the RCA reagents are not present earlier. In an embodiment, the RCA reaction is initiated by adding at least a polymerase enzyme.

The method is particularly suited to the homogenous, in solution, format. Accordingly, in an embodiment, the method is performed in solution, that is in the absence of a solid phase, at least up to step (v). In the words, in an embodiment, the padlock probe contacting step, the silencing oligonucleotide contacting step, the padlock probe hybridisation and ligation steps and the RCA steps are performed in solution, or in the absence of a solid phase.

In an advantageous embodiment, the method is performed in a single reaction vessel, e.g. a single tube.

As will be discussed in more detail below, the target nucleic acid molecule may be an analyte, or it may be a nucleic acid molecule which is used or generated as a proxy, or a reporter or indicator, for the analyte to be detected.

In an embodiment the silencing oligonucleotide is pre-hybridised to, premixed with, or added to the sample together with, the padlock probe. In other embodiments, the silencing oligonucleotide is contacted with the sample after the padlock probe has been allowed to hybridise to the target nucleic acid molecule, or after it has been ligated.

In an embodiment, the silencing oligonucleotide comprises a third silencing region, which lies between the first and second silencing regions, such that the silencing oligonucleotide binds to the padlock probe at three sites.

In another aspect also provided herein is a kit for detecting a target nucleic acid molecule in a sample, said kit comprising:
(i) a padlock probe which comprises at its 5' and 3 'ends target-binding regions which are complementary to probe-binding sites in the target nucleic acid molecule;
(ii) a silencing oligonucleotide which comprises first and second silencing regions which are complementary to the target-binding regions at the ends of the padlock probe, wherein the two silencing regions are separated from one another by at least 5 nucleotides; and
(iii) a strand-displacement polymerase.

As indicated above, the silencing oligonucleotide may optionally comprise a third silencing region. Thus, the stretch of nucleotides (i.e. the nucleotide sequence) which separates the first and second silencing regions can be capable of hybridizing to the backpiece of the padlock or not. The backpiece is the part of the padlock between the target-binding regions.

In this aspect the padlock probe and silencing oligonucleotide may be as defined above.

In still another aspect, provided herein is a reagent comprising a silencing oligonucleotide for blocking the target binding regions of a padlock probe and the padlock probe hybridised to the silencing oligonucleotide, said silencing oligonucleotide comprising first and second silencing regions which are complementary to the target-binding regions at the ends of the padlock probe, wherein the two silencing regions are separated from one another by at least 5 nucleotides, and wherein the silencing oligonucleotide further comprises a third silencing region, which lies between the first and second silencing regions and which is complementary to a silencing oligonucleotide-binding site in the padlock probe which lies between the target binding regions of the padlock probe. The silencing oligonucleotide-binding site is separated from the target-binding regions in the padlock probe by sequences thar are not complementary to the silencing oligonucleotide

### Description of Figures

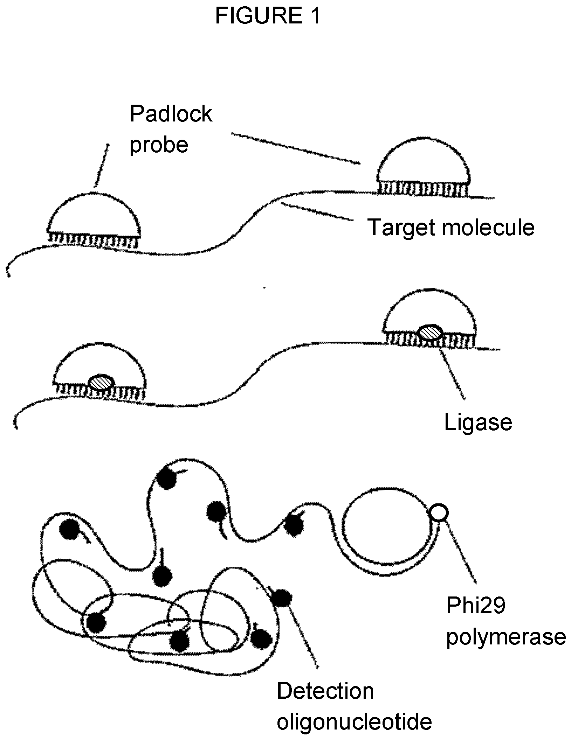

Figure 1 depicts a reaction scheme for detection of a target nucleic acid molecule using padlock probes. The target binding regions at the 5' and 3' ends of the probes are depicted hybridised directly adjacent to one another on the target nucleic acid molecules. The two ends are ligated to one another using a ligase enzyme, forming a circular template which is amplified by RCA using Phi29 polymerase, to generate a long linear concatemeric RCP. The RCP is detected using labelled detection oligonucleotides.

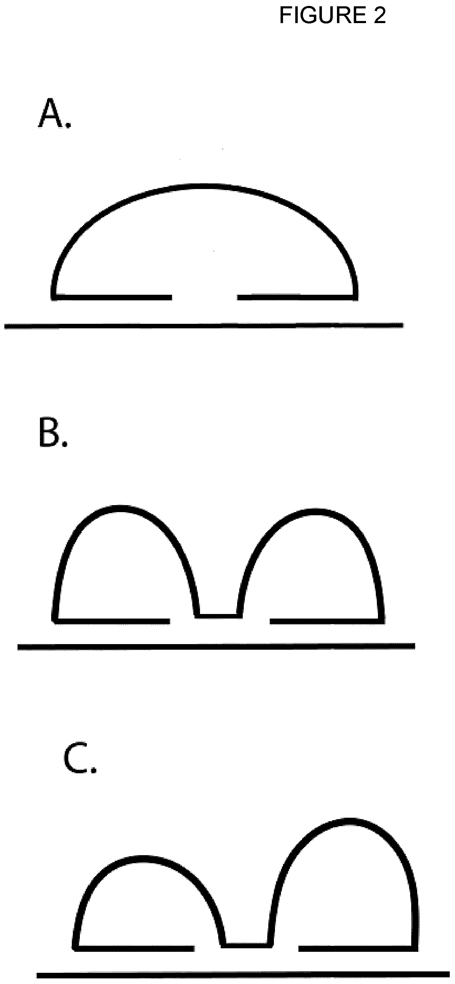

Figure 2 depicts hybridisation of a padlock probe to a silencing oligonucleotide; (A) silencing oligonucleotide with two silencing regions, each complementary to an end of the padlock probe, the two silencing regions being separated by a sequence which is not complementary to the padlock probe, such that there is a gap between the hybridised ends of the probe; (B) and (C) alternative configurations of the hybridisation of a padlock probe to a silencing oligonucleotide with a third silencing region which hybridises to an intermediate part of the padlock probe (the "backbone" or "backpiece"), lying between the two end regions.

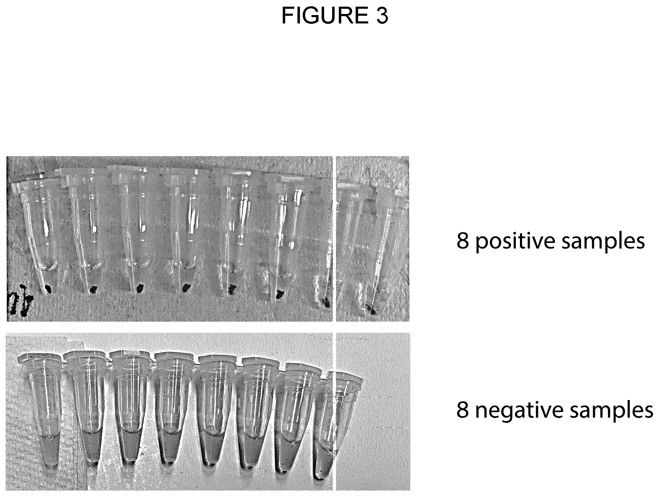

Figure 3 shows the results of an experiment with the model system as described in Example 1. A synthetic template comprising 9 different target sequences is added to a set of tubes (referred to as "positive samples"). A control set of tubes (referred to as "negative samples") does not comprise the synthetic template, and comprises a synthetic non-target template molecule (referred to as an "incorrect template"). A ligation mix comprising 9 padlock probes targeting the 9 target sequences, 9 cognate silencing oligonucleotides, one for each padlock probe, and ligase is added to the tubes, and incubated. An RCA mix comprising reagents for RCA and detection beads is added and RCA is performed. The top panel shows 8, repetitive assays of the same, positive samples and the bottom panel shows 8, repetitive assays of the same, negative samples.

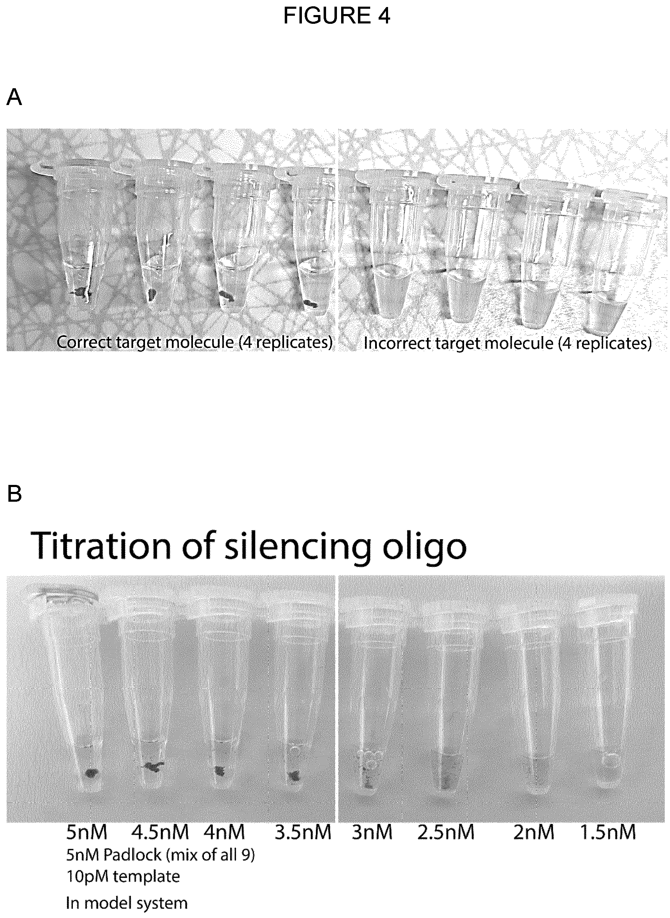

Figure 4 shows (A) the results of an experiment with the model system of Example 1 comparing the correct target molecule (synthetic template) with an incorrect template molecule; (B) titration of the silencing oligonucleotide in the model system, 5nM padlock probes, 5, 4.5, 4, 3.5, 3, 2.5, 2, 1.5 nM silencing oligonucleotides.

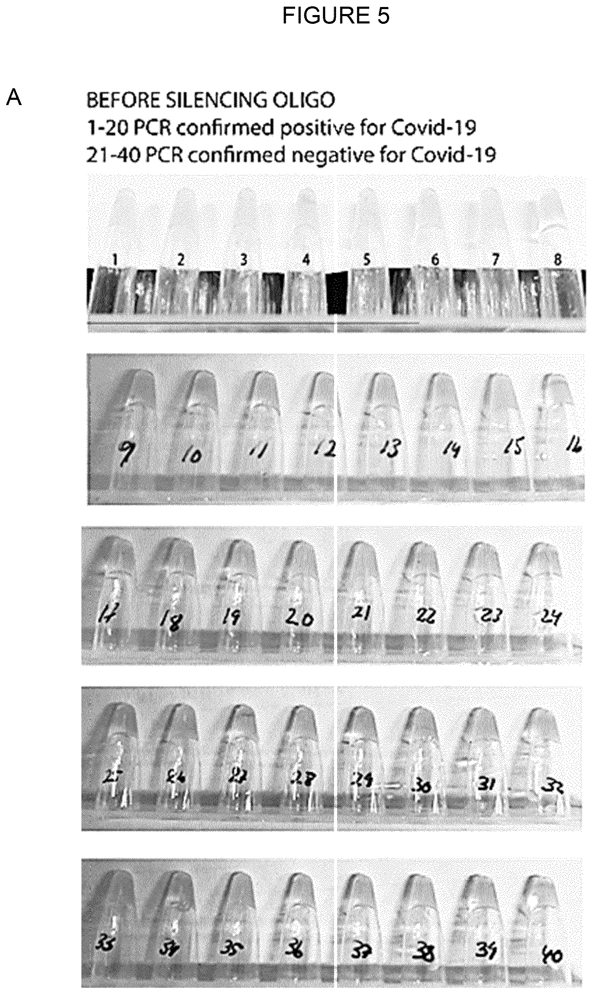

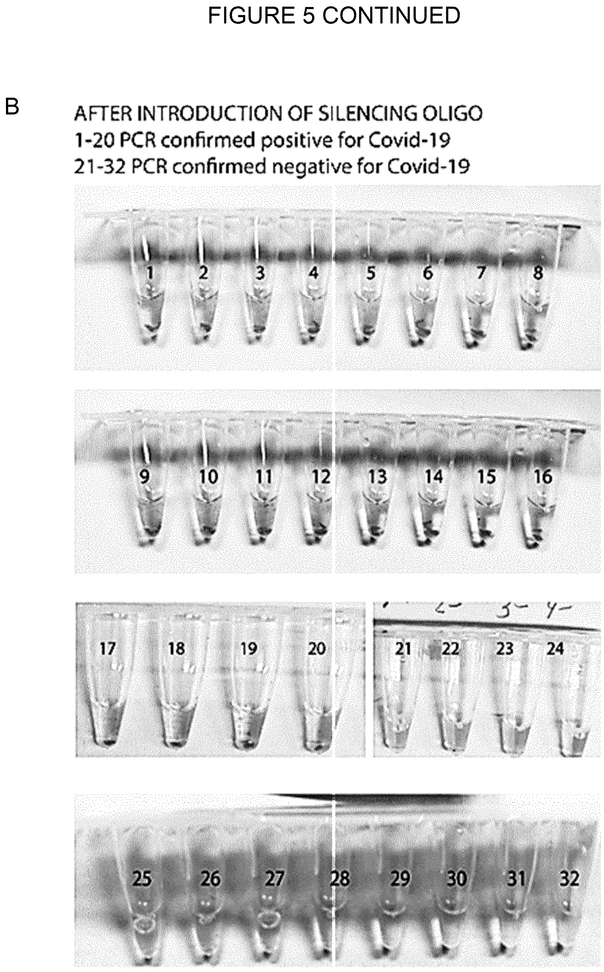

Figure 5 shows the results of testing of clinical samples. The samples in tubes 1-20 were confirmed by PCR to be positive for COVID-19 and the samples in tubes 21-40 were confirmed by PCR to be negative for COVID-19. (A) shows the results in the absence of silencing oligonucleotides and (B) shows the results of in the presence of silencing oligonucleotides.

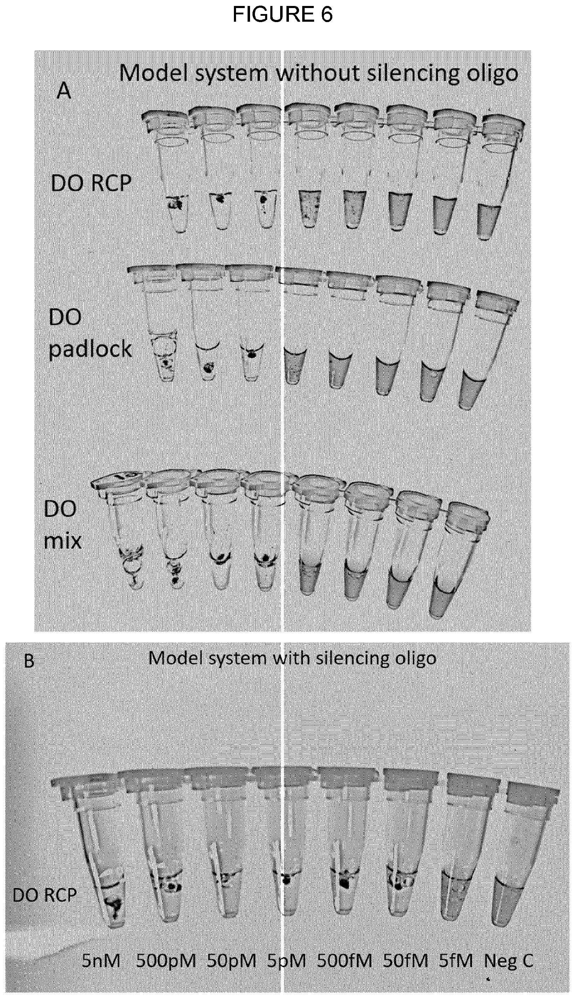

Figure 6 shows results from the model system demonstrating that increased sensitivity is obtained when using the triple-binding silencing oligonucleotide. (A) is a titration of the template oligonucleotide in the model system without the silencing oligonucleotide. (A) shows the template oligonucleotide at concentrations of 5nM, 500pM, 50pM, 5pM, 500fM, 50fM, 5fM and negative control (Neg C), and shows binding of different detection oligonucleotides to either the rolling circle product (RCP) or the free padlock, that is not in this experiment inhibited by the silencing oligonucleotide from interacting with the RCP. The detection limit here is between 5pM and 50pM. (B) is a titration of the template oligonucleotide in the model system together with the triple-binding silencing oligonucleotide. (B) shows the template oligonucleotide at concentrations of 5nM, 500pM, 50pM, 5pM, 500fM, 50fM, 5fM and Neg C, together with the silencing oligonucleotide at a concentration of 1:1 ratio to the padlock. Shown here is that the silencing oligonucleotide allows the RCP to grow without interference of a free padlock binding with one arm to the growing padlock. If the padlock is allowed to bind with one of its arms, the RCP will become double stranded. Only a perfect binding of the padlock to the growing RCP is possible (since the padlock prefers to bind a perfect target (18+18nt (36nt)) than to the triple-binding silencing oligo; however, if the free padlock binds the RCP with one arm, the silencing oligo will then bind the other arm and the phi29 cannot make the product double stranded) which then allows for a second-generation RCP. The silencing oligonucleotide allows for perfect binding of the padlock to the template which increases the sensitivity of the assay down to a limit of detection between 5fM and 50fM.

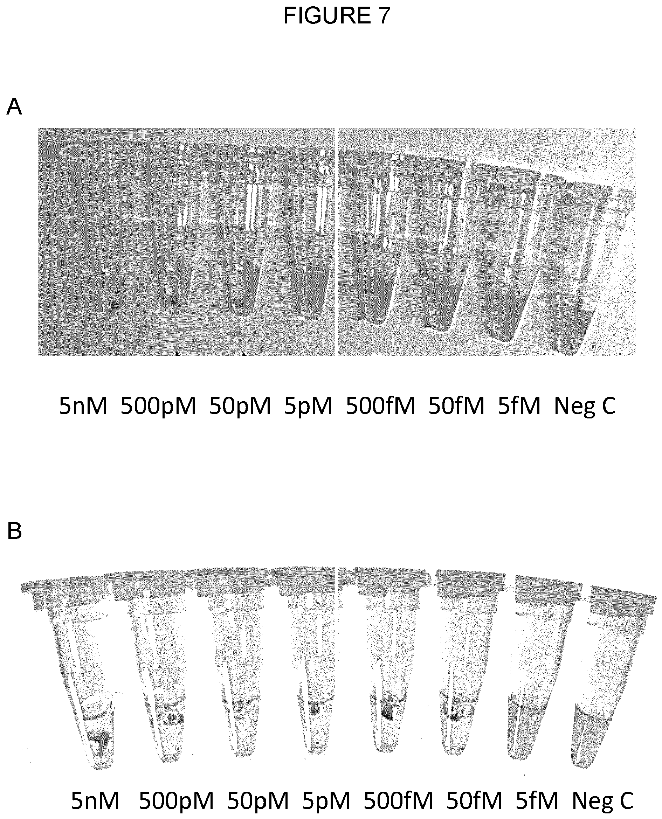

Figure 7 presents data from the model system comparing 2 designs of the silencing oligonucleotide, at different template oligonucleotide concentrations of 5nM, 500pM, 50pM, 5pM, 500fM, 50fM, 5fM and Neg C, together with the silencing oligonucleotide at a concentration 1:1 ratio to the padlock. The "double-binding" silencing oligonucleotide in (A) has two silencing regions (binding 2 positions) compared to the "triple-binding oligonucleotide in (B) which has three silencing regions (binding 3 positions). The results show that a silencing oligonucleotide with 3 silencing regions allows for a second round of RCA (on top of the first round RCP). This only occurs when the silencing oligonucleotide binds the padlock at 3 positions (B). The silencing oligonucleotide that does not bind the backpiece of the padlock (A) does not allow for this lower sensitivity - it does not allow for the second round of RCA. The silencing oligonucleotide with 3 binding sites on the padlock allows for perfect binding of the padlock to the template which increases the sensitivity of the assay to a limit of detection between 50fM and 5fM compared to a limit of detection between 50pM and 5pM when using the 2 binding sites.

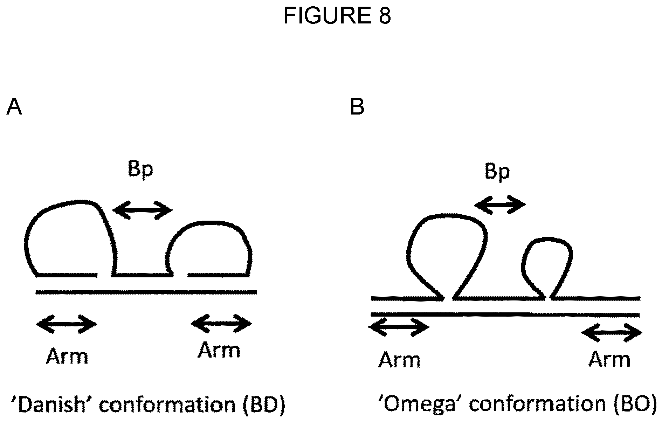

Figure 8 depicts two different designs for a silencing oligonucleotide having 3 silencing regions (binding sites for the padlock probe) - (A) the "Danish" conformation (BD) and (B) the "Omega" conformation (BO). The first and second silencing regions binding to the target-binding regions at the ends of the padlock probe are referred to as "arms" and the third silencing region binding to the intermediate (backpiece) part of the padlock probe is referred to as Bp ("backpiece").

### Detailed description

The present method provides an improved method for detecting a target nucleic acid molecule in a sample. More particularly, the method may be used to detect a specific nucleotide sequence, or in other words a target nucleotide sequence, in a target nucleic acid molecule (the term "nucleotide sequence" may be used interchangeably with "nucleic acid sequence").

The method is based on detecting the target nucleic acid using a padlock probe, which upon binding to its target is ligated, and subjected to a RCA reaction. The product of the RCA reaction is detected in order to detect the padlock probe, and thereby the target nucleic acid. The improvement relates to an improved method for removing, or eliminating free padlock probes, which have not hybridised and become ligated. The free padlock probes are eliminated by rendering them inert, by blocking them, using a silencing oligonucleotide such that they cannot participate in any non-specific or unwanted reactions, which interfere with the assay and/or which generate non-specific signals. In this way, the specificity and/or the sensitivity of the assay method herein may be improved. Thus, the "removal" or "elimination" herein does not require a physical removal in the sense of separation from the sample, but rather includes a functional removal or elimination. When silenced, or blocked, by the silencing oligonucleotide the padlock probe cannot be ligated, or is substantially not available for ligation. Further it cannot, or substantially is not able to, prime an extension reaction on any other nucleic acid template which may be present or available in the sample, since when hybridised to the silencing oligonucleotide it is not available, or is substantially not available, to hybridise to any other nucleic acid molecule which might serve as a template for RCA.

As will be described in more detail below, in a padlock probe-RCA-based detection method the RCA product (RCP) may typically and conveniently be detected by hybridising detection oligonucleotides to the RCP, which detection oligonucleotides are detected in the RCP. To allow the detection oligonucleotides to bind to the RCP it must remain in the single-stranded form in which it was generated by RCA of the circularised padlock probe. Thus, it is important to prevent unwanted extension reactions from occurring using the RCP as an extension template, which would render the RCP double-stranded. It is thus undesired for free padlock probes which did not hybridise to the target nucleic acid molecule, or padlock probes which were not ligated in the previous step, to be available to hybridise to the RCP and to prime an extension reaction thereon.

Accordingly, in an embodiment, the purpose of the silencing oligonucleotide is effectively to bind to any free unbound, or unligated, padlock probes present in the reaction mixture (the mixture resulting from contacting the sample with the padlock probes, and allowing the probes to become hybridised, or hybridised and ligated), and in this way to sequester them. Thus, in this embodiment, the silencing oligonucleotide acts to prevent, or to reduce or inhibit, any free padlock probes present in the reaction mixture, that is padlock probes which have not hybridised to the target nucleic acid, or have not been ligated, from hybridising to, or being able to hybridise to, the RCP.

The silencing oligonucleotide is accordingly contacted with the padlock probe before the RCA is performed (i.e. before the RCA reaction is initiated).

However, whilst it is generally not desired in RCA reactions for unreacted padlock probes to bind to the RCP, in so-called super RCA (sRCA) reactions, a padlock probe is used to bind to a first generation (or first round) RCP in order to perform a second round RCA reaction using the first RCP as a ligation template for a padlock probe, leading to the generation of a second generation RCA product by RCA of the ligated padlock on the first RCP (to generate a so-called "second RCP"). We have found that certain designs of silencing oligonucleotide, namely those having three silencing regions, may facilitate, or enable, such second round RCA reactions (or in other words, sRCA). This is described in more detail below.

In certain embodiments, the silencing oligonucleotide/padlock probe cognate pair may be designed so as to minimise any possible displacement of the silencing oligonucleotide from its hybrid with the padlock probe by the RCP once it is generated. Further, by including detection oligonucleotides in the RCA reaction mix, these may hybridise to the RCP as it is generated, thereby minimising the risk of any such possible displacement. Further, the silencing oligonucleotide/padlock probe hybrid may itself hybridise to the RCP, via another part of the padlock probe. Such a bound padlock would not be able to be amplified by RCA.

In other embodiments, the silencing oligonucleotide/padlock probe cognate pair may be designed so as to favour binding of the padlock probe to its target (or "template") sequence, such that in the presence of the target sequence, the padlock probe will bind to the target sequence rather than the silencing oligonucleotide. This is the case in sRCA reactions, where a second round of RCA is desired.

By "removing" or blocking unreacted padlock probes in this manner, the method lends itself particularly well to homogenous assays, that is assays which are performed in solution, without a solid phase. However, its use in the context of heterogenous, or solid-phase based, methods is not precluded.

As noted above, the use of padlock probes and RCA to detect target nucleic acids according to the method herein is known in the art. The operation of such a detection method is illustrated schematically in Figure 1. The padlock probe hybridises to the target nucleic acid. As will be discussed in more detail below, depending on the configuration of the padlock probe, the target-binding regions of the padlock probe may be capable of hybridising to (or in other words, be complementary to) the target sequence in the target molecule that it is desired to detect. Alternatively, the target binding regions of the padlock probe may be capable of hybridising (or be complementary) to binding sites in the target nucleic acid molecule which flank the target sequence. This latter option may be the case, for example, when the padlock probe is a gap-filling padlock, wherein the gap between the hybridised 5' and 3' ends of the padlock probe is filled by polymerase-catalysed extension of the hybridised 3' end of the padlock, using the target nucleic acid molecule as an extension template. The padlock probe is contacted with the target nucleic molecule in the sample and allowed to hybridise to the target molecule.

The term "contacting" is used broadly herein to include any means of bringing the sample, or more particularly, the target nucleic acid molecule, into contact. This may involve for example, adding the padlock probe to the sample, and holding, e.g. incubating, the resulting reaction mixture under conditions which allow the probe to hybridise to the target nucleic acid molecule. Alternatively, the sample, or an aliquot or portion thereof, may be added to the padlock probe, or to a reaction mix comprising the padlock probe.

Upon hybridisation of the padlock probe to the target nucleic acid, the ends of the padlock probe are brought into juxtaposition for ligation to each other, directly if the ends are hybridised directly adjacent to one another, or indirectly if the ends of the padlock probe are hybridised with a gap between them. As mentioned above, and described in more detail below, the gap may be filled by a gap oligonucleotide, or by gap-filling extension. As depicted in Figure 1, the ends of the padlock probe hybridise directly adjacent to one another, such that they can be directly joined by ligation. The padlock probe is circularised by the ligation, thereby generating a circular template for the RCA reaction. The RCA reaction generates a long concatemeric product (the RCP) comprising multiple repeats of a complementary copy of the circularised padlock probe, joined in tandem. In other words, the RCP comprises multiple monomers, joined to one another, each monomer representing a complementary copy of the circularised padlock probe. As depicted in Figure 1, the padlock probe comprises a detection sequence which is copied into each monomer repeat in the RCP, and which provides a binding site for a detection oligonucleotide. Hybridisation of the detection oligonucleotides to their multiple binding sites, concentrates them in the RCP, allowing it to be detected with high sensitivity. As described in more detail below, the detection oligonucleotide may be provided with a detectable moiety, e.g. a label, which allows it to be detected. As depicted in Figure 1, the label is a coloured bead, but any detectable label may be used.

Any padlock probes which have failed to hybridise to the target, or which have failed to become ligated, may be, or may become, free in the sample, or more particularly in the reaction mixture. To prevent such unbound or unligated padlock probes from being able, or available, to hybridise to the RCP which is generated, and to prime unwanted extension reactions, before the RCA reaction is initiated the silencing oligonucleotide is used to "capture" or "block" such probes. More particularly, the target binding regions of the padlock probe are blocked by binding to the silencing oligonucleotide, for example as depicted in Figure 2.

At its most general, the method is for detecting a target nucleic acid molecule. More particularly the method is for detecting a target nucleic acid sequence in a target nucleic acid molecule. The term "detecting" is used broadly herein to to include any means of determining the presence of the target nucleic acid molecule. In the present method the target nucleic acid is detected by detecting the presence or amount of the RCP which is generated, and can include detecting simply if it is present or not, or any form of measurement of the RCP. Thus, the RCA product generated in step (v) may be detected as the "signal" for the target nucleic acid molecule. Accordingly, detecting the RCP in step (vi) includes determining, measuring, assessing or assaying the presence or absence or amount or location of the RCP in any way. The presence of a RCP (i.e. the confirmation of its presence or amount) is indicative or identificatory of the presence of the target nucleic acid molecule, as the successful generation of the RCP is dependent on the presence of the target nucleic acid molecule (or more particularly the presence of a target nucleic acid sequence therein).

Quantitative and qualitative determinations, measurements or assessments are included, including semi-quantitative. Such determinations, measurements or assessments may be relative, for example when two or more different target nucleic acid sequences, or target molecules, in a sample are being detected, or absolute. Accordingly, in an embodiment the method may be for quantifying or determining the amount of target nucleic acid molecule or sequence which is present. The term "quantifying" when used in the context of quantifying a target nucleic acid molecule(s) or sequence(s) in a sample can refer to absolute or to relative quantification. Absolute quantification may be accomplished by inclusion of known concentration(s) of one or more control nucleic acid molecules and/or referencing the detected level of the target nucleic acid molecule or sequence with known control nucleic acid molecules or sequences (e.g. through generation of a standard curve). Alternatively, relative quantification can be accomplished by comparison of detected levels or amounts between two or more different target nucleic acid molecules, or different target sequences, to provide a relative quantification of each of the two or more different nucleic acid molecules or sequences, i.e., relative to each other. Thus, as noted above, ratios of target nucleic acid molecules or sequences present in sample may be determined. Thus, for example, copy numbers of target nucleic acid molecules, e.g. chromosomes, may be compared.

The target nucleic acid molecule is any nucleic acid molecule it is desired to detect, or in other words the target of the assay. More particularly, the target may be a target sequence present in the nucleic acid molecule. It may in some embodiments be desirable to detect two or more target sequences which are present in a target molecule. In other embodiments, two or more target molecules, or two or more target sequences present in two or more target molecules may be detected. The method may be performed in multiplex to detect two or more different target sequences, for example, in one or more target nucleic acid molecules, and/or a target sequence in two or more target molecules. Thus, to detect target sequences in two or more different target molecules, a multiplicity of first padlock probes may be used, each specific for a different target sequence, that is having target-binding regions which are complementary to binding sites in the target sequence, or which for example flank the different target sequences. It will be understood in this respect that where the target binding sites flank a target sequence in the molecule, the flanking binding sites in the different target molecules may be different for different target sequences, to allow for specific binding of the padlock probes. Thus, in such a situation the flanking sequences which are targeted by the padlock probes are discriminatory between different targets. They may thus be regarded as part of the target sequence. Alternatively, or additionally, different and separate target sequences in the same target molecule may be detected, for example, different sequences in different genes on a chromosome, again using a multiplicity of different padlock probes, each specific for a different target sequence.

In a particular embodiment, the method is useful for detecting which of a number of possible different variant sequences is present in a given target molecule, for example whether a wild-type or mutant sequence is present, or which of a number of possible mutants, or different allelic variants, or polymorphisms etc. In such a protocol, the padlock probes may comprise target binding regions which are designed to discriminate between different variants. Alternatively, a gap-fill padlock probe may be used which hybridizes to flanking regions which flank all or multiple variants, and a gap-fill extension step is carried out, which generates a complementary copy of the variant sequence(s) that is (are) present. The nature of the variant sequence may be determined, or the variant may be detected, by detecting the complement of the variant sequence that is present in the RCP. This may be for example, by sequencing or by using a detection oligonucleotide which is specific for the variant sequence.

The method may thus be used to detect multiple target molecules or target sequences. The term "multiple" as used herein means two or more, for example, 3, 4, 5, 6, 8, 9, 10, 15, 20, 25, 30, 40, 50, 60, 70, 80, 90, or 100 or more. Indeed, thousands or tens of thousands of padlock probes may be used. The number of padlocks that can be used is not restricted, and can be varied. This will depend on the purpose of the method, the nature of the sample, and the target nucleic acid sequences to be detected, number of possible variants etc. Thus, to detect wild-type and mutant variants for examples, the number of different padlocks will depend on the number of different mutants possible, and may be for example, 2-6, 2-5, 2-4 or 2-3 different padlocks. It will be appreciated that different aspects may be combined, in order to increase the overall multiplex of the assay. For example, in a given sample, the method may be used to detect different variants of different target sequences.

The target nucleic acid molecule or sequence may be any molecule or sequence it is desired to detect or identify. It may be DNA or RNA, or a modified variant thereof. Thus, the nucleic acid may be made up of ribonucleotides and/or deoxyribonucleotides as well as synthetic nucleotides that are capable of participating in Watson-Crick type or analogous base pair interactions. Thus, the nucleic acid may be or may comprise, e.g. bi-sulphite converted DNA, LNA, PNA or any other derivative containing a non-nucleotide backbone.

Typically, the target molecule or sequence will be an analyte it is desired to detect, for example a nucleic acid present in a sample, e.g. in a cell or tissue sample, or any biological or clinical sample etc. It may be a viral nucleic acid. Thus, it may be a naturally occurring sequence, or a derivative or copy or amplicon thereof. However, the method is not restricted to this, and the target molecule may instead be a reporter for an analyte of an assay. Reporter nucleic acids may be used or generated in the course of an assay for any analyte, for example a protein or other biological molecule, or small molecule, in a sample. Thus, a reporter nucleic acid may be provided as a tag, or label, for a binding probe for an analyte, and may be detected in order to detect the analyte, for example in an immunoassay, e.g. as in an immunoPCR or immunoRCA reaction. A reporter nucleic acid may be generated in the course of an assay, for example by a ligation reaction in a proximity ligation assay, or an extension reaction in a proximity extension assay, or by a cleavage reaction, or such like. Such a reporter target nucleic acid may therefore be a synthetic or artificial sequence.

In an embodiment, the target nucleic acid is a DNA molecule, natural or synthetic. The target nucleic acid molecule may be coding or non-coding DNA, for example genomic DNA or a sub-fraction thereof, or may be derived from genomic DNA, e.g. a copy or amplicon thereof, or it may be cDNA or a sub-fraction thereof, or an amplicon or copy thereof etc.

In another embodiment, the target nucleic acid molecule is a target RNA molecule. It may be an RNA molecule in a pool of RNA or other nucleic acid molecules for example genomic nucleic acids, whether human or from any source, from a transcriptome, or any other nucleic acid (e.g. organelle nucleic acids, i.e. mitochondrial or plastid nucleic acids or viral nucleic acids), whether naturally occurring or synthetic. The target RNA molecule may thus be or may be derived from coding (i.e. pre-mRNA or mRNA) or non-coding RNA sequences (such as tRNA, rRNA, snoRNA, miRNA, siRNA, snRNA, exRNA, piRNA and long ncRNA). In one embodiment, the target nucleic acid molecule is a micro RNA (miRNA). In one embodiment, the target RNA molecule is 16S RNA, for example wherein the 16S RNA is from and identificatory of a microorganism (e.g. a pathogenic microorganism) in a sample. Alternatively, the target RNA molecule may be genomic RNA, e.g. ssRNA or dsRNA of a virus having RNA as its genetic material. Notable such viruses include Ebola, HIV, SARS, SARS-CoV2, influenza, hepatitis C, West Nile fever, polio and measles. Accordingly, the target RNA molecule may be positive sense RNA, negative sense RNA, or double-stranded RNA from a viral genome, or positive-sense RNA from a retroviral RNA genome.

Where the target molecule is an RNA molecule, the method may comprise a preliminary step of generating a cDNA copy of the target RNA molecule. The cDNA molecule is then contacted with the padlock probe in step (i).

Alternatively, a target RNA molecule may directly be contacted with the padlock probe. In other words, the first padlock probe may bind directly to the target RNA molecule.

For the detection of a variant target sequence, as described above, the padlock probe may be specific for a particular variant. However, as also mentioned above, this is not a requirement, and in an alternative embodiment a padlock probe is used which is capable of capturing any possible variant of a given target sequence. Such a padlock probe is thus not selective, or specific, for any particular variant. However, to ensure capture of a desired target sequence it will be designed to specifically bind to the target molecule at sites which allow all variants of the target sequence to be captured, that is at sites which flank the target sequence, and which are common between different variants. By way of example, the variant sequence may be a mutation or a polymorphism at a particular position or locus in a gene. The target sequence may thus be a sequence which includes or comprises that variant position or locus, and different target sequences may be distinguished by having different bases at that position or locus. The padlock probe may be designed to have binding sites which are complementary to flanking sequences in the target molecule which are shared or conserved (i.e. common) between the different variants.

Accordingly, in such an embodiment, the padlock probe may be viewed as a common probe, or as generic to a group of target sequences, or a group of variant target sequences. The padlock probe may thus have target-binding regions which are complementary to binding sites (i.e. flanking regions) in the target molecule which are common to different target sequences (that is common binding sites which flank different target sequences, or different variants of a target sequence). Alternatively put, the padlock probe may have target binding regions which are common, or generic, for different target sequences, or different variants, or common for a group of target sequences or group of variants. That is the padlock probe may have target binding regions which are capable of hybridising to complementary binding sites in the target molecule which are common to different target sequences, or to different variants of target sequences.

In a different embodiment, the padlock probe may be specific for a particular target nucleic acid sequence. Thus, for example different padlock probes may be used, each specific for a different target sequence. This may have utility for diagnostic testing, e.g. NIPT, where different sequences may be detected, for example to detect different chromosomes (and determine their copy number, for example to detect a trisomy) or in any situation where it is desired to detect one or more specific target sequences.

A padlock probe may alternatively be defined as a circularisable probe. The use of padlock or circularisable probes is well known in the art, including in the context of RCA reactions. A circularisable probe comprises one or more linear oligonucleotides which may be ligated together to form a circle. Padlock probes are well known and widely used and are well-reported and described in the literature. Thus, the principles of padlock probing are well understood and the design and use of padlock probes is known and described in the art. A padlock probe is typically a linear circularisable oligonucleotide which hybridizes to its target nucleic acid sequence or molecule in a manner which brings 5' and 3' ligatable ends of the probe into juxtaposition for ligation together, either directly, or as described above, indirectly, with a gap in between. By ligating the hybridized 5' and 3' ends of the probe, the probe is circularized. It is understood that for circularization (ligation) to occur, the ligatable 5' end of the padlock probe has a free 5' phosphate group.

To allow the juxtaposition of the ends of the padlock probe for ligation, the padlock probe is designed to have the target-binding sites at its 5' and 3' ends. That is, the regions of complementarity which allow binding of the padlock probe to its target lie at the ends of the padlock probe.

To allow ligation, the 3' and 5' ends which are to be ligated (the "ligatable" 3' and 5' ends) are hybridized to the target molecule or sequence, which acts as the ligation template. The binding of the padlock probe brings the ends into said juxtaposition. Where the complementary binding sites in the target molecule or sequence lie directly adjacent (or contiguous) to one another, the ends of the padlock probe will hybridise directly adjacent to each other (i.e. with no gap) and may be ligated to each other directly. Thus, in this case the ligatable ends of the probe are provided by the actual ends of the probe. In the case of a gap-fill padlock probe, the target binding regions at the ends of the padlock probe do not hybridise to adjacent binding sites, but rather to non-adjacent (non-contiguous) binding sites in the target molecule. In such an arrangement, the 5' ligatable end of the probe is provided by the actual 5' end of the probe. However, the ligatable 3' end of the probe is generated by extension of the hybridized 3' end of the probe, using the target sequence as extension template to fill the gap between the hybridized ends of the probe. The extension reaction brings the extended 3' end of the probe into juxtaposition for ligation. In this case, the ligatable 3' end of the probe is thus the extended 3' end of the probe.

Padlock probes may be provided in 2 or more parts that are ligated together. This may involve the provision of an additional ligation template, for example in the case of a 2-part probe, where each part comprises only one target-binding region, and the other end of each part hybridizes to a common ligation template. In another embodiment, a 2-part padlock may take the form of a "connector" or "backbone" oligonucleotide with two target-binding regions at or near the 5' and 3' ends respectively, which hybridise to the target with a gap in between them, and a gap oligonucleotide which hybridizes in the gap between the ends. The gap oligonucleotide may partially or fully fill the gap. In the case of a gap-fill padlock probe, the 3' end which is extended may be the hybridized 3' end of the gap oligonucleotide or the backbone oligonucleotide.

In a typical embodiment, however, the padlock is provided as a single circularisable oligonucleotide, whether as a gap fill padlock, or not.

In particular embodiments the padlock probe does not have secondary structure, and more particularly does not comprise intramolecular double-stranded regions or stem-loop structures. However, dumbbell probes, which do have secondary structure, are a particular sub-type of padlock probe. The dumbbell probe comprises two stem-loop structures, joined stem to stem, wherein one of the "loops" is not closed, but is open with free 5' and 3' ends available for ligation to each other. This "open loop" functions as the target-binding domain of the probe. The closed loop functions simply as a spacer to join the end of the duplex (stem). In other words, it can be seen as a padlock probe with a region of duplex formed between complementary sequences (regions) of the padlock. The region of duplex functions as a signalling domain to which an intercalating agent can bind. Thus the "open loop" of a dumbbell probe may comprise the target-binding regions of complementarity.

The padlock probe may comprise one or more other regions or sequences which may be useful in the method. This may include in particular a detection sequence which allows the probe to be detected, or identified. A detection sequence may for example be an identificatory sequence, such as a bar-code sequence which may be detected to detect the padlock probe (more particularly, the complement of the detection sequence in the RCP may be detected). Alternatively, the detection sequence, or more particularly its complement, may be detected by means of a detection oligonucleotide, which hybridises to the complement of the detection sequence in the RCP. The detection oligonucleotide is thus a detection probe, which is detected in order to detect the RCP, and thereby the padlock probe which was used to generate it, thereby detecting the target nucleic acid molecule. The detection oligonucleotide may be provided with a detection moiety as discussed further below. A complementary copy of the detection sequence in the padlock probe is generated in each monomer repeat in the RCP. This complementary copy in the RCP is capable of hybridising to the detection oligonucleotide and may thus itself be complementary to the detection oligonucleotide. It can thus be seen that the detection sequence in the padlock probe may correspond to the sequence of the detection oligonucleotide which is used to hybridise to the RCP.

A target sequence to be detected in the method may comprise one or more variant bases. Thus, it may be a single nucleotide variant, e.g. a single nucleotide polymorphism (SNP) or mutation, or it may comprise two or more bases. Thus, a variant sequence may comprise a stretch of nucleotides, 2 or more bases of which may be variant. The variant bases may be contiguous or non-contiguous.

The length of the target sequence is not critical, and may vary according to circumstance, and the nature of the target molecule, the target sequence, or the variant position or locus. A target sequence may thus be, by way of representative example only, 1 to 10, e.g., 1-15, 1-12. 1-10, 1-8, 1-7 or 1-6 nucleotides long. In certain embodiments however, a target sequence longer than a single nucleotide may be beneficial, to improve specificity, and in such embodiment, the target sequence may be from any one of 2, 3, 4, 5, or 6 to any one of 6, 7, 8, 9, 10, 12, 15 or 20 nucleotides long. Exemplary target sequences may thus be 4-10, 4-8, 4-7, 4-6, 5-10, 5-8, 5-7, or 6-8 nucleotides long for example.

The silencing oligonucleotide is a linear oligonucleotide comprising at least two, that is at least first and second, silencing regions. The only requirement is that the silencing regions are capable of hybridising to the target binding regions of the padlock probe which lie at the ends of the padlock probe, for example as depicted in Figure 2, such that there is a gap of at least 5 nucleotides between the ends of the padlock probe when hybridised to the silencing oligonucleotide. Accordingly, the silencing regions are separated in the silencing oligonucleotide by a gap sequence.

In particular the gap sequence between the silencing regions may be at least 6, 7, 8, 9 or 10 nucleotides long.

By way of example, the gap sequence between the silencing regions may, be 5-50, 6-50, 7-50, 8-50, 9-50, 10-50, 5-40, 6-40, 7-40, 8-40, 9-40, 10-40, 5-30, 7-30, 8-30, 10-30, 5-25, 7-25, 8-25, 10-25, 5-20, 7-20, 8-20 or 10-20 nucleotides long.

In an embodiment, the silencing regions may lie at the 5' and 3' ends of the silencing oligonucleotide, but this is not a requirement.

The silencing oligonucleotide may comprise further silencing regions which are complementary to other sequences in the padlock probe, which lie between the ends of the padlock probe, that is to sequences which are internal in the sequence of the padlock probe. In particular, the silencing oligonucleotide may comprise a third silencing region. This is depicted in Figures 2 B and C, and in Figure 8. The third silencing region lies between the first and second silencing regions in the silencing oligonucleotide. It is complementary to a silencing-oligonucleotide binding site in the padlock probe which typically may lie between the 5' and 3' ends of the padlock probe. Such a silencing oligonucleotide is referred to herein as "triple binding" or as a "triple-binder" and represents a particularly advantageous aspect.

For example, the presence of the third silencing region, when hybridised to the padlock probe, may prevent the hybridised 3' end of the padlock probe from being extended by the polymerase which is added for the RCA reaction.

It will thus be understood the third, or further, silencing region lies within the gap sequence. It may correspond to the gap sequence in its entirety, or to a part of the gap sequence. Thus, the gap sequence may be, or may comprise a sequence which is complementary to a sequence in the padlock probe which is not a target binding region of the probe. In such a configuration, the third silencing region may be complementary to any part of the padlock probe which is not a target-binding region. This silencing oligonucleotide-binding site typically lies between the target binding regions at the 5' and 3' ends of a padlock probe, or of the backbone part of a 2-part padlock probe. The third silencing region may be referred to as binding to a backpiece part of the padlock probe, or as being a backpiece silencing region.

In an embodiment, the third silencing region may be capable of hybridising to (e.g. may be complementary to) all or part of a detection sequence of the padlock probe. In other words, the detection sequence may fully or partially overlap with the silencing oligonucleotide-binding site which is complementary to the third silencing region of the silencing oligonucleotide.

Alternatively, where the silencing oligonucleotide does not comprise any further silencing regions beyond the first and second silencing regions, the gap sequence is not complementary to any part of the padlock probe. The gap sequence may for example be a random sequence.

As noted above, the triple-binding configuration of the silencing oligonucleotide can allow a second round of RCA to be performed using the RCP that is generated in the first round (i.e. the "first RCP") as the ligation template for the padlock probe. This is demonstrated in Figure 7, and described in the Examples below. It has been found that a triple-binding silencing oligonucleotide, having first and second silencing regions which bind to the first and second target binding regions of the padlock probe (also referred to herein as the "arms" of the padlock probe), and a third silencing region binding to the padlock backpiece) may disfavour the binding of the padlock to silencing oligonucleotide relative to binding of the padlock to its target sequence. In other words, in such a configuration, the padlock probe may prefer to bind to its target sequence rather than the silencing oligonucleotide. In the absence of target sequence, or where the padlock-binding sites in the target sequence are blocked or occupied, the silencing oligonucleotide will bind to the padlock probe. However, in the presence of the target sequence (also referred to as "template sequence") the padlock probe will bind to the target sequence. Whilst not wishing to be bound by theory, it is hypothesised that this may be due to the padlock-target sequence binding structure being less rigid than the padlock-silencing oligonucleotide binding structure.

The target-binding sites (arms) of the padlock probe are complementary to the target sequence. The first RCP that is generated by RCA of the ligated padlock probe comprises monomers that are complementary to the padlock probe, and it will therefore be seen that the first RCP comprises, within its monomer repeats, repeating homologous copies of the target sequence. Thus, these repeating copies are copies of the target sequence and provide binding sites for the padlock probe. In the presence of ligase, it has been shown that, due to the preferential binding of the padlock probe to the target sequence, the padlock probe may be ligated and serve as a template for a second round of RCA to generate a second-generation RCA product (a "second RCP"). This results in signal amplification. The first RCP comprises multiple repeated binding sites for the padlock probe, and therefore multiple second RCPs can be generated. Such sRCA reactions are known in the art.

The skilled person can readily design cognate silencing oligonucleotide/padlock probe pairs for use in the RCA or sRCA reaction as desired. It is known how to design oligonucleotides which differing hybridisation strengths, and accordingly to achieve favoured hybridisation for a desired hybridisation binding pair.

The lengths of the silencing regions of the silencing regions are not critical as long as they are long enough to achieve a specific hybridisation to its complementary binding site in the padlock probe. Again, they may be varied according to design, and the selected configuration of binding to the padlock probe. The first and second silencing regions bind to the target-binding sites of the padlock probe. Whilst they need not correspond exactly to the target binding sites, they must be long enough to bind specifically to those regions. A first and second silencing region is typically at least 6 nucleotides long. A third silencing region may be shorter, for example at least 2 nucleotides long. Various different lengths have been investigated and found to be effective, as described in the Examples below.

In representative embodiments, the first and second silencing regions may be from at least any one of 6, 7, 8, 9, or 10 nucleotides long. The maximum length is not critical, and will depend on the length of the target binding sites of the padlock probe. For example, they may be up to any of one of 7, 8, 9, 10, 11, 12, 15, 20, 25, or 30 nucleotides long, or more if desired. Representative ranges include any range between any of the above minimum or maximum integers, e.g. 6-30, or 6-20, or 6-18. Other ranges include 6-15, 6-12, 7-20, 8-20, 9-20, 10-20 etc. The third silencing region may for example be 2-50, 2-40, or 2-30 nucleotides long,

Different combinations of lengths of first/second and third silencing regions may be used. For example, first and second silencing regions of 18 nt may be used in combination with a third silencing region of 20 nt, and have been shown to work well, but this may be varied for use with a shorter third silencing region. Likewise, first and second silencing regions of 10 nt also work well with a third silencing region of 20 nt.

As noted above, the first and second silencing regions need not correspond exactly in length to the target binding regions of the probes, and thus the complementary binding site in the padlock probe for the silencing region may be shorter or longer than the binding site to the target sequence of the padlock probe. A first or second silencing region of 10-12nt in length may for example be shorter than the target binding site of the padlock probe and may for example leave a stretch of nucleotides at the ends of the padlock probe free (i.e. the 3'- and/or 5'- most nucleotides of the probe, namely those closest to the ligation site).

A silencing oligonucleotide is provided for each padlock probe that is used. In other words, each padlock probe has a cognate silencing oligonucleotide. Thus, for example, where different target specific padlock probes are used, each comprising different target binding regions, each padlock probe will have a different cognate silencing oligonucleotide. However, in the case where a single or "common" padlock probe is used, only a single cognate silencing oligonucleotide will be needed.

To perform the method the sample, or more particularly the target nucleic acid, is contacted with the padlock probe. This contact may include other reagents. The sample containing the target nucleic acid molecule, or a part or fraction or aliquot thereof may be contacted with the padlock probe. For example, for *in situ* analyses, or indeed for other analyses, for example of clinical samples, a sample may be contacted with the reagents directly. The sample may be prior-treated or processed prior to the contact, e.g. by fixation of a cell or tissue sample. In other embodiments the target nucleic acid may first be separated, or removed from the sample. Procedures for extracting or purifying nucleic acids, e.g. DNA, from various types of sample are well known in the art. For example, the nucleic acids may be isolated from cells, or from cell-free samples, such as plasma. It may in some cases also be desirable to fragment nucleic acid molecules. Procedures for this are known in the art, and include specific digestion, e.g. using nucleases, including restriction enzymes for example, or by non-specific means, such as shearing.

The contacting step with the padlock probe prepares a reaction mixture for the probe-binding step. However, reagents for subsequent steps may also be included, for example for an extension step if the padlock is a gap-fill padlock. Further, in another embodiment, or additionally, reagents may be included for the ligation reaction. Generally speaking, the initial reaction mixture will not include all the reagents necessary to perform the RCA reaction, to allow the RCA reaction to be controlled (i.e. initiated later). However, in some embodiments it may be possible for some RCA reagents (e.g. RCA primer, or nucleotides) to be included with the padlock probes, or more generally in the initial reagent mixture which is contacted with the sample.

To perform the probe-binding step the padlock probe is typically incubated with the target nucleic acid molecule. For a gap-fill padlock, dNTPs and polymerase may also be included. Conveniently a ligase may also be included during the probe binding steps. The reagents may be added in a single reaction mix, or separately before or during the probe-binding step. To allow for probe-binding there may be an initial heating step, for example to denature a double-stranded nucleic acid molecule.

The reagents are typically provided in a buffer, according to principles and procedures known in the art. For example, a buffer appropriate for the selected ligase enzyme may be selected.

The reaction mixture may be incubated in conditions appropriate to facilitate or enable padlock probe binding (the so-called "annealing" step). If there has been a preceding denaturation step this may involve a reduction in temperature. Conditions for these steps are known in the art, and are within the routine skill of the skilled practitioner in the art to select or design. For example, an annealing temperature of room temperature, or in the range of 20-40 °C may be used, e.g. 25-40 °C, or 25-37 °C. In on the embodiments a higher temperature, e.g. 50-65 °C may be used, e.g. 53 to 60 °C, or 55 to 60 °C.

If an elevated annealing temperature is selected, the annealing temperature may be reduced for the extension step if a gap-fill padlock is used. Again, the appropriate conditions can be selected according to what is known in the art, and the particular reagents, e.g. enzymes used. For example, after the initial annealing step, the temperature may be reduced to 28-40 °C, e.g. 28-35, 30-35, 28-33, 30-33, 28-33, or 30-32 °C etc.

The silencing oligonucleotide is contacted with the padlock probe before, during or after the sample is contacted with the padlock probe, but before the RCA reaction takes place (i.e. before the RCA reaction is initiated).

Conveniently, the silencing oligonucleotide may be contacted with the sample at the same time as the padlock probe. In this regard, the silencing oligonucleotide may be contacted separately or in admixture. Thus, in an embodiment, a reagent mix may be prepared which comprises the padlock probe and the silencing oligonucleotide. In another embodiment, the reagent mix may further comprise a ligase for the padlock probe ligation step. The mix may be contacted with the sample. Alternatively, reagents may be added individually to the sample. The resulting reaction mixture may then be incubated, to allow padlock probe binding and ligation to take place.

The silencing oligonucleotide may be provided in a form where it is pre-hybridised to the padlock probe, and such a reagent is contacted with the sample, or used to prepare the initial reagent mix.

It will be understood in this regard that in such a format where the silencing oligonucleotide is pre-hybridised, or is added to the sample together with the padlock probe, the target nucleic acid displaces the silencing oligonucleotide from the padlock or out-compete the silencing oligonucleotide from binding to the padlock probes. In others words, hybridisation of the padlock probe to the target nucleic acid is favoured over hybridisation to the silencing oligonucleotide. However, any padlock probes which have not hybridised to the target molecule remain hybridised to, or become hybridised to, the silencing oligonucleotide.

Alternatively, in another embodiment, a reagent mix may be prepared comprising the padlock probes and a ligase enzyme and this may be contacted with the sample, and the padlock probes allowed to hybridise to the target nucleic acid. The silencing oligonucleotide may then be added. Any unbound padlock probes present in the reaction mixture are allowed to hybridise to the silencing oligonucleotide.

In another embodiment, the padlock probes may be contacted with and allowed to hybridise to the target nucleic acid. Ligase may then be added and allowed to ligate any padlock probes that have become bound. Subsequently, the silencing oligonucleotide may be added, to hybridise to any padlocks which have not hybridised, and which have not been ligated.

Where the padlock is a gap-fill padlock, the initial reaction mix for padlock binding may also include a polymerase and nucleotides for the extension step. In such a procedure, it may be desirable to include a heat inactivation step for the polymerase enzyme, before RCA is initiated.

The padlock probe is ligated to circularise it, and thereby generate the template for the first RCA reaction. The ligation is templated by the target nucleic acid molecule. Any convenient ligase may be employed, and representative ligases of interest include, but are not limited to, temperature sensitive ligases such as SplintR ligase (also known as PBCV-1 DNA ligase or Chlorella virus DNA ligase) bacteriophage T4 DNA ligase, bacteriophage T7 ligase, and *E. coli* ligase, and thermostable ligases such as Taq ligase, Tth ligase, Ampligase^{®}, Pfu ligase and 9°N^{™} DNA Ligase.

Suitable conditions for ligation are known in the art, and any reagents that are necessary and/or desirable may be combined with the reaction mixture and maintained under conditions sufficient for ligation. It will be evident that the ligation conditions may depend on the ligase enzyme used in the methods of the invention. Thus, for example, Ampligase may be used, and the temperature may be increased for the ligation step. Alternatively, SplintR ligase may be used at room temperature.

Where temperature change or temperature control steps are required, the method may be performed in a thermal cycling instrument. This permits a ready control of the temperature changes. However, an advantage of the method is that extreme temperature changes are not necessary, and the method may for example be performed at room temperature, or at 20-37 °C, for example. Probe binding and ligation steps may for example be performed at room temperature.

The conditions for the probe binding and ligation reactions may be optimised by routine experimentation according to principles known in the art. Thus, temperature, buffers, time of incubation, ramping speed etc. may be adjusted to find the optimal conditions.

Once the padlock probe has been ligated, it is subjected to the RCA reaction. Before the RCA reaction there may be an optional washing step, for example if the method is performed in a heterogenous or solid phase format, e.g. when the sample is fixed or immobilised on a solid support. However, a benefit of the present method is that a washing step is not required. Accordingly, the inclusion of such a step is not favoured. In an embodiment, the method proceeds to the RCA step without a washing step.

The RCA reaction is then initiated. This may be achieved a reagent to the reaction mixture, in order to allow the RCA reaction to start. This can be a complete RCA reaction mix, comprising all the reagents required for the RCA reaction (e.g. polymerase and nucleotides, and optionally a primer. Alternatively, one or more reagents may be added. For example, a single reagent may be added. Conveniently, the RCA reaction may be initiated by adding polymerase. The polymerase is conveniently added by adding a RCA reagent mix comprising other reagents for the RCA reaction, for example nucleotides, in an appropriate buffer. However, in some embodiments other reagents for the RCA reaction may be added or included earlier.

As noted above, RCA reactions are well known in the art, and hence the conditions for this step may be designed or selected according to protocols and principles known and described in the literature. A strand-displacing polymerase enzyme such as Phi29 or derivatives thereof is used.

The primer for the RCA reaction may be added to the reaction mixture, or may be pre-hybridised to the padlock probe. The binding site for the RCA primer may be provided in a region of the padlock probe which is different to the target binding regions (i.e. in the backbone region of the padlock). In some cases the target nucleic acid molecule may serve as or provide the primer. If necessary, 3' exonuclease action of the polymerase, or a separate exonuclease may be used to digest the target nucleic acid to provide a hybridised 3' end suitable to act as the RCA primer for RCA of the circularised padlock probe.

The RCA reaction produces a concatemeric RCA product (RCP) comprising multiple repeat copies of the complement of the padlock probe. The RCP is detected to detect the target nucleic acid molecule.

The method may be carried out in heterogenous or homogenous formats. That is, it may be performed on a solid phase (or support), or in solution or suspension (i.e. without a solid phase or support), or indeed both, since a solid phase may be introduced at a later stage, for example at the step of detecting the RCP.

The format of the method may be selected based on the nature of the sample, or the target nucleic acid molecule, or the desired readout or detection technology used. For example, for liquid or liquefied or solubilised samples, or for isolated or purified nucleic acids etc., or for the detection of non-nucleic analytes in samples (for example detection of proteins in serum or plasma, or other body fluids etc.), or indeed for detecting any analyte in convenient diagnostic or clinical tests, a solution phase format may be adopted.

On the other hand, to detect target sequences in solid samples, such as tissues or cells, for example for *in situ* detection, a solid phase format may be used. It may also be desired to immobilise the sample or target nucleic acid for other reasons, e.g. for a particular assay format, or simply according to choice. Thus, a sample of isolated cells may be fixed on support, or nucleic acids may be isolated or captured from a sample onto a solid support, or such like. In another embodiment, the primer for the second RCA reaction may be immobilised on solid support.

In one embodiment the method may be for the localised detection of target nucleic acid molecules, "Localised" detection means that the signal giving rise to the detection of the nucleic acid is localised to the nucleic acid, in this case the RCP is localised to the target nucleic acid. The nucleic acid may therefore be detected in or at its location in the sample. In other words the spatial position (or localization) of the nucleic acid within the sample may be determined (or "detected"). This means that the nucleic acid may be localised to, or within, the cell in which it is located or expressed, or to a position within the cell or tissue sample. Thus "localised detection" may include determining, measuring, assessing or assaying the presence or amount and location, or absence, of nucleic acid in any way.

In a particular embodiment, the method may be used for the localised, particularly *in situ,* detection of a target nucleic acid sequence. More particularly, the method may be used for the localised, particularly *in situ,* detection of a nucleic acid, particularly mRNA, in a sample of cells.

As used herein, the term *"in situ"* refers to the detection of a target nucleic acid sequence in its native context, i.e. in the cell or tissue in which it normally occurs. Thus, this may refer to the natural or native localization of a target nucleic acid sequence, e.g. RNA. Typically, this term refers to the nucleic acid as it occurs within a cell or within a cell or tissue sample, e.g. its native localization within the cell or tissue and/or within its normal or native cellular environment

In other embodiments, as noted above, the detection is not localized, or not *in situ.* In still other embodiments, the method can be carried out in solution. In particular the nucleic acid can be in solution. Thus, for example, the method can be performed on a sample comprising isolated nucleic acid.

The target nucleic acid molecule is present in the sample. The sample may be any sample which contains any amount of nucleic acid, from any source or of any origin, in which it is desired to detect a target nucleic acid molecule. A sample may be any clinical or non-clinical sample, and may be any biological, clinical or environmental sample in which the target nucleic acid molecule may occur.

The sample may be any sample which contains a target nucleic acid molecule, and includes both natural and synthetic samples, that is, materials which occur naturally or preparations which have been made. Naturally occurring samples may be treated or processed before being subjected to the methods herein. All biological and clinical samples are included, e.g. any cell or tissue sample of an organism, or any body fluid or preparation derived therefrom, as well as samples such as cell cultures, cell preparations, cell lysates etc. Environmental samples, e.g. soil and water samples or food samples are also included. The samples may be freshly prepared or they may be prior-treated in any convenient way e.g. for storage.

Representative samples thus include any material which may contain a target nucleic acid molecule, including for example foods and allied products, clinical and environmental samples. The sample may contain any viral or cellular material, including all prokaryotic or eukaryotic cells, viruses, bacteriophages, mycoplasmas, protoplasts and organelles. Such biological material may thus comprise all types of mammalian and non-mammalian animal cells, plant cells, algae including blue green algae, fungi, bacteria, protozoa etc., or a virus. The cells may be for example human cells, avian cells, reptile cells etc., without limitation.

Representative samples thus include whole blood and blood-derived products such as plasma, serum and buffy coat, blood cells, urine, faeces, cerebrospinal fluid or any other body fluids (e.g. respiratory secretions, saliva, milk, etc.), tissues, biopsies, cell cultures, cell suspensions, conditioned media or other samples of cell culture constituents, etc. The sample may be pre-treated in any convenient or desired way to prepare for use in the method, for example by cell lysis or purification, isolation of the nucleic acid, etc.

In one embodiment the sample comprises microbial cells or viruses which have been isolated from a clinical sample or from a culture of a clinical sample. In such a sample the target nucleic acid molecule may be a nucleotide sequence present in a microbial cell or virus, e.g. a nucleotide sequence which is characteristic for, or discriminatory or identificatory of a microbial cell or virus, at any level, e.g. at type, group, class, genus, species or strain level.

In another embodiment the sample may contain cell-free DNA. The sample may be sample such as plasma or serum which directly contains cell-free DNA, or the cell-free DNA may be isolated. The cell-free DNA may comprise circulating tumour DNA.

For localised *in situ* detection the sample may be any sample of cells in which a nucleic acid molecule may occur, to the extent that such a sample is amenable to localized *in situ* detection. This may be a sample in which the nucleic acid is present at a fixed, detectable or visualizable position in the sample. The sample will thus be any sample which reflects the normal or native *("in situ")* localization of the nucleic acid, e.g. RNA, i.e. any sample in which it normally or natively occurs. Such a sample will advantageously be or comprise a cell or group of cells such as a tissue. Examples are samples such as cultured or harvested or biopsied cell or tissue samples in which the nucleic acid may be detected to reveal the qualitative nature of the nucleic acid, i.e. that it is present, or the nucleotide sequence of the nucleic acid or the presence and/or identity of one or more nucleotides in the nucleic acid, and localization relative to other features of the cell. The sample of cells may be freshly prepared or may be prior-treated in any convenient way such as by fixation or freezing. Accordingly, fresh, frozen or fixed cells or tissues may be used, e.g. FFPE tissue (Formalin Fixed Paraffin Embedded). The sample may comprise any cell type that contains nucleic acid including all types of cells as discussed above.

The sample may also be treated to fix nucleic acid, e.g. RNA, contained in the cells to the sample, for example to fix it to the cell matrix. Such procedures are known and described in the art. For example, in the field of *in situ* hybridization, reagents are known for fixing mRNA to cells. In particular, 5' phosphate groups in the RNA may be linked to amines present on proteins in the cellular matrix via EDC-mediated conjugation (EDC: 1-ethyl-3-(3-dimethylaminopropyl) carbodiimide), thus helping to maintain the localization of the RNA relative to other cellular components. Such a technique has previously been described in relation to microRNAs and their detection via in situ hybridization. Alternatively, in procedures where a reverse transcription step is performed to generate cDNA from a RNA target, the 5' end of the cDNA primer and/or cDNA molecules can be subject to crosslinking activity. This may be achieved using for example DSP chemical or Acrylic acid NHS ester.

Since the target nucleic acid molecule need not itself be the target analyte of the assay, but can for example be a reporter molecule used or generated in the course of an assay for any desired analyte, the sample need not be a sample which naturally contains nucleic acid, or a source of nucleic acid (e.g. a cell or virus, or biological or clinical material etc.). As indicated above, the sample may be a synthetic or artificial sample. It may accordingly be a sample which has been subjected to a detection assay for an analyte in which a target nucleic has been generated, or to which a target nucleic acid molecule has been added. It may be a reaction mixture, or a reaction product, for example the product resulting from an immunoassay to detect a target analyte, e.g. an immunoPCR, immunoRCA, or proximity assay (e.g. proximity ligation assay (PLA) or proximity extension assay (PEA), as discussed above.

The target analyte may be any analyte it is desired to detect. As discussed above, in embodiments the target nucleic acid molecule of the method herein is the target analyte. In other embodiments, where the target nucleic acid molecule is a reporter, the target analyte may be any analyte it is desired to detect. The analyte may be a nucleic acid, a protein (which term includes peptides and polypeptides), or any other chemical or biological molecule or moiety, including for example carbohydrates, e.g. such as may occur as glycosyl groups on proteins. The target analyte may thus be a modified protein, for example with a post-translational modification which is detected in an assay for an analyte.

In an embodiment, the target analyte may be a protein or component of a proteinaceous molecule which is detected on the surface of a cell, or vesicle, or other cellular or sub-cellular compartment. For example, extracellular vesicles, or exosomes, may be detected and distinguished by virtue of different proteins present on their surface.

As noted above, the padlock probes may comprise one or more further sequences which may serve to introduce a sequence into the ligated product, and thereby into the RCP (as a complementary copy). This may be, for example, a detection sequence, e.g. a barcode or identificatory motif, or a binding site for a detection probe or primer. Such a further sequence may be found, for example, in a portion of the backbone region of the padlock probe, that is the region between the target-binding regions. In a dumbbell probe it may be in the duplex region of the probe. Tags such as barcodes or probe/primer binding sites may be designed with different needs/purposes, for example, to introduce a universal or common sequence to enable different ligated probes in a multiplex setting to be processed together, e.g. to introduce a binding site for a universal or common amplification primer. This would enable different ligated probes to be amplified together, e.g. in a library amplification by PCR or RCA.

In particular, the padlock probe may contain a detection sequence by which it may be detected. A complement of the detection sequence will become incorporated into the RCP, and may be detected, for example by the binding to it of a detection probe, or by sequencing. The detection sequence may be specific to the padlock probe, and thus to the target sequence, or sequence variant it is desired to detect. Thus, each padlock probe may have a different detection sequence. The detection sequence may be detected to detect or identify which padlock probe was amplified in the RCA, and hence which target sequence was present. Such a protocol may be applied, for instance, in the context of a method for detecting a target sequence variant, where each s padlock is provided with a detection sequence specific to particular variant. The detection sequence may thus be seen as a marker or identification sequence. The term "detection sequence" as used herein includes both the detection sequence as it occurs in the padlock probe, and the complementary copy as it appears in the RCP.

Accordingly, a detection sequence, may be used to "tag" or mark different padlock probes so that they, or their ligation or amplification products may readily be distinguished from one another. Additionally, such a sequence may be used to tag different samples etc. for example so that they may be pooled (i.e. a "sample" tag or marker). Thus, in a multiplex setting, different probes (i.e. probes for different target nucleic acid sequences or different variants) may be provided with different tag sequences (e.g. different marker or detection sequences) and/or they may be provided with the same tag sequence(s) e.g. for the introduction of a common or universal sequence. Such methods may be used in conjunction with particular detection methods, including the use of detection probes or sequencing methods such as sequencing by hybridization, sequencing by ligation or other next generation sequencing chemistries, e.g. in the multiplexed detection of multiple target nucleic acids in a sample.

The term "hybridisation" or "hybridises" as used herein refers to the formation of a duplex between nucleotide sequences which are sufficiently complementary to form duplexes via Watson-Crick base pairing, or any analogous base-pair interactions. Two nucleotide sequences are "complementary" to one another when those molecules share base pair organization homology. Hence, a region of complementarity in a molecule or probe or sequence refers to a portion of that molecule or probe or sequence that is capable of forming a duplex. Hybridisation does not require 100% complementarity between the sequences, and hence regions of complementarity to one another do not require the sequences to be fully complementary, although this is not excluded. Thus, the regions of complementarity may contain one or more mismatches. Accordingly, "complementary", as used herein, means "functionally complementary", i.e. a level of complementarity sufficient to mediate a productive hybridisation, which encompasses degrees of complementarity less than 100%. The degree of mismatch tolerated can be controlled by suitable adjustment of the hybridisation conditions. Those skilled in the art of nucleic acid technology can determine duplex stability empirically considering a number of variables including, for example, the length and base pair composition of the respective molecules or probe oligonucleotides, ionic strength, and incidence of mismatched base pairs, following the guidance provided by the art. Thus, the design of appropriate probes, and binding regions thereof, and the conditions under which they hybridise to their respective targets is well within the routine skill of the person skilled in the art.

A region of complementarity, such as for example to a target sequence in the binding region of a padlock probe, or between a detection sequence and a detection oligonucleotide, or a RCA primer to the circularised padlock probe etc., may be at least 6 nucleotides long, to ensure specificity of binding, or more particularly at least 7, 8, 9 or 10 nucleotides long. The upper limit of length of the region is not critical, but may for example be up to 50, 40, 35, 30, 25, 20 or 15 nucleotides. A complementary region may thus have a length in a range between any one of the lower length limits and upper length limits set out above. In the case of a padlock probe, the length of an individual target-binding region may be in the lower ranges, so that the total length of the two binding regions when hybridised to their target is within the upper ranges. For example, an individual target binding region may be 8-15, e.g. 10-12 nucleotides, so that the total hybridised length is 16-30 nucleotides long, e.g. 20-24. It may be desirable, within the constraints of conformation of the probes, and spacing of the domains, and desired or favoured hybridisations, to minimise the total length of a padlock probe to minimise the size of the circle which is subjected to RCA, and hence to minimise the lengths of the complementary regions where possible.

The RCP may be detected using any convenient protocol. Depending on the target molecule to be detected, the purpose of the method, and/or the specific details of the procedures employed in the method, the detection protocol employed may detect the RCP non-specifically or specifically.

For instance, the RCP may be detected directly, e.g. the concatemer may be cleaved to generate monomers which may be detected using gel electrophoresis, or more typically by hybridizing labelled detection oligonucleotides that hybridize to the detection sequence in the RCP, as discussed above. The detection oligonucleotide need not, however, be directly labelled. For example, the detection oligonucleotide may be an unlabelled probe which functions as a sandwich probe. The concept of sandwich probes is well known in the art and may be applied according to any convenient protocol. The sandwich probes can bind to the RCP but are not directly labelled themselves; instead, they comprise a sequence to which labelled secondary oligonucleotides can bind, thus forming a "sandwich" between the RCP and the labelled secondary oligonucleotide.

A RCP may also be detected using non-sequence-specific nucleic acid labelling methods, e.g. DNA binding stains or dyes, which are widely known in the literature, or by using labelled nucleotides for incorporation into the RCP. Alternatively, the RCP may be detected indirectly, e.g. the product may be amplified by PCR and the amplification products may be detected.

The RCP may be detected using any of the well-established methods for analysis of nucleic acid molecules known from the literature including liquid chromatography, electrophoresis, mass spectrometry, including CyTOF, microscopy, real-time PCR, fluorescent probes, microarray, colorimetric analysis such as ELISA, flow cytometry, mass spectrometry, or by turbidometric, magnetic, particle counting, electric, surface sensing, weight-based detection techniques. Generally speaking, such techniques are relevant for in solution assays.

Advantageously, however, the RCP may generally be detected using labelled detection oligonucleotides, that is detection oligonucleotides which are provided with a detection moiety. The detection moiety is any moiety which can be detected, that is which can give rise, directly or indirectly to a signal which can be detected. The detection moiety may thus be viewed as any detectable label, which may be directly or indirectly signal-giving. For example, the detection moiety may be spectroscopically or microscopically detectable, e.g. it may be a fluorescent or colorimetric label, a particle, e.g. a bead or an enzymatic label. Any of the labels used in immunohistochemical techniques may be used.

In multiplex procedures for detecting different target sequences and/or variant target sequences different RCPs products may be detected and distinguished by *in situ* sequencing, including for example sequencing by synthesis, sequencing-by-hybridisation and sequencing by ligation, next generation sequencing and/or sequential barcode decoding techniques, and/or by using detection probes oligonucleotides. Depending on the level of multiplexing, combinatorial labelling methods may be used, according to techniques well known in the art, for example, via ratio labelling with fluorescent or other spectrophotometrically detectable oligonucleotides. Such ratio-labelled detection probes may be used during flow cytometry, or microscopic detection techniques, e.g. imaging, to detect large numbers of sequences, e.g. the combination of at least two fluorophores at different ratios can lead to generation of multiple populations of fluorescent labels.

In methods which involve the use of detection oligonucleotides, the detection oligonucleotide or any secondary labelling probe may be labelled with a directly or indirectly detectable label. A directly detectable label is one that can be directly detected without the use of additional reagents, while an indirectly detectable label is one that is detectable by employing one or more additional reagents, e.g., where the label is a member of a signal producing system made up of two or more components. In many embodiments, the label is a directly detectable label, where directly detectable labels of interest include, but are not limited to: fluorescent labels, coloured labels, radioisotopic labels, chemiluminescent labels, and the like. In many embodiments, the label is a fluorescent label, where the labelling reagent employed in such embodiments is a fluorescently tagged nucleotide(s), e.g. fluorescently tagged CTP (such as Cy3-CTP, Cy5-CTP) etc. Fluorescent moieties which may be used to tag nucleotides for producing labelled probe nucleic acids (i.e. detection probes) include, but are not limited to: fluorescein, the cyanine dyes, such as Cy3, Cy5, Alexa 555, Bodipy 630/650, and the like. Other labels, such as those described above, may also be employed as are known in the art.

Conveniently for detection in a solution phase method, the detection moiety may be a coloured bead. Coloured beads may readily be visualised. Such beads, e.g. coloured polystyrene beads, are widely available.

Although various detection modalities may be employed, conveniently the RCPs may be detected by visualisation, including by microscopy, or flow cytometry. In both cases directly or indirectly labelled detection oligonucleotides may be used, for example with fluorescent or coloured labels which may readily be detected. In this regard the label may include a bead or other detectable particle. In a microscopy-based method, the RCPs may be detected by imaging.

As noted above, also provided herein are kits for performing the methods. The kits may include the padlock probes and silencing oligonucleotides as discussed above, optionally together with one or more reagents and/or instructions for use of the kit. Such reagents include dNTPs, and polymerase and ligase enzymes, as well as an RCA primer for the RCA reaction. Further, components may include buffers or other reaction components for one or more of the various reactions. Still further optional components may include means or reagents for detecting the RCP. This may include for example, detection oligonucleotides and any necessary secondary labelling reagents, including for example as discussed above. Further optional components may include a solid support and/or means for capture and/or immobilisation of a target nucleic acid molecule, or of a reaction component. Instructions may be for example in printed form, or on a computer-readable medium, or as a website address.

As noted above, the method may be performed using a solid phase, for example, in which the RCA product becomes immobilised on a solid phase. This may result from immobilisation of the target molecule, for example in *in situ* detection procedures.

Immobilisation of the first RCA product and/or target molecule on a solid phase may be achieved in various ways according to principles well known in the art. Accordingly, several embodiments of solid phase assays are contemplated. In one such embodiment, the sample may be provided on a solid support, such an in an *in situ* application for example. Alternatively, the target nucleic acid molecule may be captured by an immobilised (or immobilisable) capture probe, and the RCA product can be generated such that it is attached to the analyte, for example by virtue of the primer for the RCA being the target molecule or being attached to the target molecule. Alternatively, the RCA product may simply be immobilised to a solid support. For example, the primer for the first RCA may be provided with an immobilisable group or moiety or means for immobilisation, or may be immobilised, prior to the first RCA.

As noted above, the target nucleic acid may itself may be immobilised (or immobilisable) on the solid phase e.g. by non-specific absorption. In a particular such embodiment, the molecule may be present within cells, being optionally fixed and/or permeabilised, which are (capable of being) attached to a solid support, e.g. a tissue sample comprising the target molecule may be immobilised on a microscope slide.

Advantages of the methods herein are discussed above. Such advantages are particularly beneficial in the context of facilitating the performance of in-solution assays. By avoiding the need for washing steps, or exonuclease clean-up of unhybridised probes, a simplified assay protocol may be provided. In particular, the method may readily be performed in a single reaction vessel, for example a single tube or reaction well. The method may thus be performed quickly, in a matter of minutes, by adding reagents to the reaction vessel. Further, the RCP may be detected in the single vessel, for example by using detection oligonucleotides provided with a readily visualisable detection moiety such as a coloured bead. In this manner, a straightforward visual read-out may be obtained, for example to detect the presence or absence of a target nucleic acid.

Such a method lends itself well to clinical diagnostic assays. This includes advantageously a diagnostic assay for home testing, or for use in the field, without the need for a clinical diagnostic laboratory.

By way of example, the method may be adopted for the testing of clinical samples for the presence of pathogens, e.g. by detecting the presence or absence of pathogen nucleic acid. This may include the testing of samples such as saliva or nasal or oral swabs for the presence of viruses, including notably SARS-CoV2.

Furthermore, the technique is rapid, has minimal instrument requirements, and allows multiplex analysis of sequence variants for enhanced sensitivity.

### Examples

The method will now be described in more detail in the following non-limiting examples.

### Example 1 - Model system

### Methods

1) A synthetic template was prepared representing a target SARS-CoV-2 genome comprising 9 different target sequences. The template was added to tubes at a concentration of 10 pM in 20µl of volume. Control tubes were prepared without the template, and comprising instead an "incorrect template" in the same amount. (i.e. a synthetic non-target template molecule, to represent "negative sample tubes").
2) A ligation mix was prepared by mixing 5nM of each padlock probe, 9 different padlock probes targeting different sequences of the target genome, with 1-5nM of each silencing oligonucleotide (1 silencing oligonucleotide per padlock) and ligase. 5 µl of ligation mix was added to the template. The tubes were incubated for 5 minutes at room temperature.
   1. Alternative 1. Mix Padlock and ligase and add to template. Add silencing oligonucleotide and incubate for 5 minutes in room temperature.
   2. Alternative 2. Add Padlock to the template, followed by ligase, followed by silencing oligonucleotide. Incubate for 5 minutes at room temperature.
3) A RCA reaction was performed. The ingredients for RCA were prepared by adding detection beads and Phi29 polymerase to an RCA mix comprising BSA, Phi29 buffer, dNTPs. 15µl of RCA mix was added to the ligation reaction and the tubes were incubated for 25 minutes at 37°C. The detection beads are functionalized coloured beads carrying a detection oligonucleotide.
4) The results were read by visual inspection of the tubes.

### Results

As depicted in Figure 3, sample tubes comprising the synthetic target template reported a positive result (top panel). A positive result can be seen by visualisation of a distinct coloured RCA reaction product at the bottom of the tubes (resulting from aggregation of the coloured detection beads in the RCP at the bottom of the tubes). Tubes which did not contain the synthetic target template (i.e. which contained the incorrect template) reported a negative result; no aggregation of the colored beads by the RCA reaction product can be seen and the coloured detection beads can be seen homogenously distributed throughout the reaction solution in the tubes.

To show the reproducibility of the method, the experiment was repeated comparing the synthetic template, representing the correct target nucleic acid molecule (4 replicates) with a control template lacking a target sequence for the padlock probes, representing an incorrect target molecule (4 replicates). The results are shown in Figure 4A. Positive results can be seen for the correct target molecule and negative results for the incorrect target molecule. This shows that specificity of the detection assay can be controlled using the silencing oligonucleotides.

The results of the titration of different amounts of silencing oligonucleotides are depicted in Figure 4B. This shows that at concentrations of 5, 4.5, 4 and 3.5 nM silencing oligonucleotides, a clearly distinct positive reaction may be visualised. RCA reaction product can still be seen at 3 nM but is less distinct at 2.5 nM. No clear RCA reaction product can be seen at 2 and 1.5 nM silencing oligonucleotides. This demonstrates that the presence of sufficient amounts of the silencing oligonucleotides allows the RCA reaction product to be visualised. This demonstrates that the skilled person would readily be able to determine an appropriate ratio between the padlock:silencing oligonucleotide and the template.

### Example 2 - Testing of clinical samples.

### Methods

1) Clinical samples were obtained, which had been confirmed positive or negative for COVID-19 by PCR. The samples were diluted as appropriate to the carrier buffer that they had been provided and prepared in. 20µl aliquots of sample were added to tubes. Tubes 1-20 contained samples PCR-positive for COVID-19 and tubes 21-40 contained samples PCR-negative for COVID-19.
2) A ligation mix was prepared by mixing 5nM of each padlock probe, 9 different padlock probes targeting different sequences of the target genome, with 1-5nM of each silencing oligonucleotide (1 silencing oligonucleotide per padlock) and ligase. 5 µl of ligation mix was added to the template. The tubes were incubated for 5 minutes at room temperature. As a comparison, the silencing oligonucleotides were omitted from the ligation mix.
   1. Alternative 1. Mix Padlock and ligase and add to template. Add silencing oligonucleotide and incubate for 5 minutes in room temperature.
   2. Alternative 2. Add Padlock to the template, followed by ligase, followed by silencing oligonucleotide. Incubate for 5 minutes at room temperature.
3) A RCA reaction was performed. The ingredients for RCA were prepared by adding detection beads and Phi29 polymerase to an RCA mix comprising BSA, Phi29 buffer, dNTPs. 15µl of RCA mix was added to the ligation reaction and the tubes were incubated for 25 minutes at 37°C. The detection beads are functionalized coloured beads carrying a detection oligonucleotide.
4) The results were read by visual inspection of the tubes.

### Results

Figure 5 shows the results of the experiment without silencing oligonucleotide in the ligation mix (A) and with the silencing oligonucleotides (B). It can be seen that in the absence of silencing oligonucleotides there is no difference between the COVID-19 positive tubes (tubes 1-20) and the COVID-19 negative tubes (tubes 21-40). In contrast, in the presence of the silencing oligonucleotides a positive result (presence of RCA reaction product) can be detected in the COVID-positive tubes. With regard to tubes 17-20 in (B), whilst the results were not as clear as with other tubes, aggregation of the coloured beads can be seen, indicating the presence of the RCA reaction product, in distinction to tubes 21-32.

### Example 3 - demonstration of increased sensitivity using the model system

An experiment was performed using the model system of Example 1, with or without silencing oligonucleotide.

The results are shown in Figure 6, which shows results from the model system demonstrating that increased sensitivity is obtained when using the triple-binding silencing oligonucleotide. (A) is a titration of the template oligonucleotide in the model system without the silencing oligonucleotide. (A) shows the template oligonucleotide at concentrations of 5nM, 500pM, 50pM, 5pM, 500fM, 50fM, 5fM and negative control (Neg C), and shows binding of different detection oligonucleotides to either the rolling circle product (RCP) or the free padlock, that is not in this experiment inhibited by the silencing oligonucleotide from interacting with the RCP. The detection limit here is between 5pM and 50pM. (B) is a titration of the template oligonucleotide in the model system together with the triple-binding silencing oligonucleotide. (B) shows the template oligonucleotide at concentrations of 5nM, 500pM, 50pM, 5pM, 500fM, 50fM, 5fM and Neg C, together with the silencing oligonucleotide at a concentration of 1:1 ratio to the padlock. Shown here is that the silencing oligonucleotide allows the RCP to grow without interference of a free padlock binding with one arm to the growing padlock. If the padlock is allowed to bind with one of its arms, the RCP will become double stranded. Only a perfect binding of the padlock to the growing RCP is possible (since the padlock prefers to bind a perfect target (18+18nt (36nt)) than to the triple-binding silencing oligo; however, if the free padlock binds the RCP with one arm, the silencing oligo will then bind the other arm and the phi29 cannot make the product double stranded) which then allows for a second-generation RCP. The silencing oligonucleotide allows for perfect binding of the padlock to the template which increases the sensitivity of the assay down to a limit of detection between 5fM and 50fM.

### Example 4 - demonstration of second round of RCA using Triple-binding silencing oligonucleotide

The model system of Example 1 was used to compare 2 different silencing oligonucleotide designs, firstly a silencing oligonucleotide comprising first and second silencing regions only (targeting the padlock probe target-binding sites/arms), separated by a 20 nt spacer sequence, and secondly a triple-binding silencing oligonucleotide comprising also a third silencing region.

The silencing oligonucleotides were tested at different concentrations of template molecule from 5nM to 5fM.

The results are shown in Figure 7, parts (A) and (B) respectively. It can be seen that in the presence of the triple-binding oligonucleotide, as seen in (B), greater amounts of RCA product can be seen, compared to the double-binding silencing oligonucleotide of (A), including at lower concentrations of template molecule.

The results show that a triple-binding silencing oligonucleotide with 3 silencing regions allows for a second round of RCA (on top of the first round). This only occurs when the silencing oligonucleotide binds the padlock at 3 positions, due to a more rigid conformation of the padlock probe/silencing oligo cognate, so when the correct template sequence is present, the padlock will then prefer binding the template rather than the silencing oligo. The silencing oligonucleotide that does not bind the backpiece of the padlock (A) does not form this more rigid conformation when binding the padlock probe, therefore there will not be a preference as such to the template sequence. Thus, (A) does not allow for this lower sensitivity - it does not allow for the second round of RCA. The triple-binding silencing oligonucleotide allows for perfect binding of the padlock to the template - first the template of the target molecule (such as the virus genome), and secondly the growing RCP that will contain the target sequence from the initial target molecule - which increases the sensitivity of the assay.

### Example 5 - investigation of different designs of Triple-binding silencing oligonucleotide.

Two designs of silencing oligonucleotide were tested in the model system of Example 1, as set out in Figure 8, referred to respectively as Danish conformation (BD) and Omega conformation (BO).
Different lengths of first, second, and third silencing region were tested, ranging from 6-18 for the first and second silencing regions ("arms") and 2-30 for the third silencing region ("backpiece", Bp).

The results are shown in Table 1 below:

**Table 1**

| **Name** | **Length** | **ΔG (binding to padlock) @ 25*C** |
|---|---|---|
| RNAPad18 667 | 79 | -17,32 |
| RNAseq 648-711 | 72 | -56,38 |
| 18-20-18 | 56 | -84,06 |
| 10-20-10 | 40 | -58,02 |
| 8-20-8 | 36 | -52,94 |
| 10-10-10 | 30 | -40,47 |
| 10-8-10 | 28 | -37,09 |
| 8-10-8 | 26 | -35,38 |
| 8-8-8 | 24 | -32,6 |
| 6-10-6 | 22 | -34,25 |

| | | |
|---|---|---|
| The results can be summarised as ΔG (pad-temp) < ΔG (pad-block) < ΔG (pad) -56 < ΔG (pad-block) < -17 | | |

Silencing oligonucleotides with first-second-third silencing regions of 18-20-18, or 10-20-10 (where the 10nt first and second silencing regions leave the target binding sites of the padlock closest to the ligation site free) are very efficient.

The efficiency of other variants is less, but nonetheless they work efficiently enough.

## Claims

1. A method for detecting a target nucleic acid molecule in a sample, said method comprising:
(i) contacting the sample with a padlock probe, which comprises at its 5' and 3' ends target-binding regions which are complementary to probe-binding sites in the target nucleic acid molecule;
(ii) contacting the padlock probe with a silencing oligonucleotide which comprises first and second silencing regions which are complementary to the target-binding regions at the ends of the padlock probe, wherein the two silencing regions are separated from one another by at least 5 nucleotides, and wherein said contacting occurs before, during or after contacting the sample with the padlock probe;
(iii) allowing the target-specific binding regions of the padlock probe to hybridise to the target nucleic acid molecule in juxtaposition for ligation, directly or indirectly, to each other;
(iv) ligating, directly or indirectly, the 5' and 3' ends of the padlock probe to circularise the padlock probe;
(v) after the silencing oligonucleotide has been contacted with the padlock probe, contacting the sample containing the circularised padlock probe with a strand-displacing polymerase and performing a RCA reaction using the circularised padlock probe as a RCA template to generate a RCA product (RCP);
(vi) detecting the RCP to detect the target nucleic acid sequence.

2. The method of claim 1, wherein the method is performed in solution.

3. The method of claim 1 or claim 2, wherein the target nucleic acid molecule is a target analyte, or is generated from a target nucleic acid analyte or is a reporter for a target analyte.

4. The method of any one of claims 1 to 3, wherein the padlock probe is pre-hybridised to the silencing oligonucleotide, and the target nucleic acid molecule displaces the silencing oligonucleotide from padlock probes which hybridise to the target nucleic acid molecule.

5. The method of any one of claims 1 to 3, wherein the padlock probes and silencing oligonucleotide are contacted simultaneously, or in admixture, with the sample containing the target nucleic acid molecule.

6. The method of any one of claims 1 to 3, wherein the silencing oligonucleotide is contacted with the sample after the padlock probe has been allowed to hybridise to the target nucleic acid molecule, or after the ligation step (iv), such that it hybridises to any unbound or unligated padlock probes in the sample.

7. The method of any one of claims 1 to 6, wherein the silencing regions lie at the 5' and 3' ends of the silencing oligonucleotide.

8. The method of any one of claims 1 to 7, wherein the silencing oligonucleotide comprises a third silencing region, between the first and second silencing regions, which is complementary to a silencing oligonucleotide-binding site in the padlock probe, wherein said silencing oligonucleotide-binding site lies internally in the padlock probe, between 5' and 3' ends thereof.

9. The method of any one of claims 1 to 8, wherein the padlock probe comprises a detection sequence, wherein the detection sequence allows the padlock probe, or an amplicon or reverse complement copy thereof to be detected.

10. The method of claim 9, wherein the detection sequence is a binding site for a detection oligonucleotide, or comprises a barcode sequence, optionally wherein the detection oligonucleotide is linked to a detection moiety, preferably wherein the detection moiety is a bead, a fluorescent or colorimetric label, a dye, or an enzyme substrate.

11. The method of claim 8 and any one of claims 9 to 10, wherein the detection sequence partially or fully overlaps with the silencing oligonucleotide-binding site which is complementary to the third silencing region of the silencing oligonucleotide.

12. The method of any one of claims 1 to 11, wherein the method is performed in multiplex to detect two or more target nucleic acid molecules in the sample, and wherein step (i) comprises contacting the sample with two or more padlock probes each specific for a different target nucleic acid, and a different silencing oligonucleotide is provided for each different padlock probe.

13. The method of any one of claims 1 to 12, wherein the method is performed in as single reaction vessel.

14. A kit for detecting a target nucleic acid molecule in a sample, said kit comprising:
(i) a padlock probe which comprises at its 5' and 3' ends target-binding regions which are complementary to probe-binding sites in the target nucleic acid molecule;
(ii) a silencing oligonucleotide which comprises first and second silencing regions which are complementary to the target-binding regions at the ends of the padlock probe, wherein the two silencing regions are separated from one another by at least 5 nucleotides; and
(iii) a strand-displacing polymerase.

15. The kit of claim 14, wherein the kit comprises two or more different padlock probes, each specific for a different target molecule, and two or more silencing oligonucleotides each specific for a different padlock probe.

16. A reagent comprising:
a silencing oligonucleotide for blocking the target binding regions of a padlock probe; and
the padlock probe hybridised to the silencing oligonucleotide, wherein said silencing oligonucleotide comprising first and second silencing regions which are complementary to the target-binding regions at the ends of the padlock probe, wherein the two silencing regions are separated from one another by at least 5 nucleotides, and wherein the silencing oligonucleotide further comprises a third silencing region, which lies between the first and second silencing regions and which is complementary to a silencing oligonucleotide-binding site in the padlock probe which lies between the target binding regions of the padlock probe, wherein the silencing oligonucleotide-binding site is separated from the target-binding regions in the padlock probe by sequences that are not complementary to the silencing oligonucleotide.

## Patentansprüche

1. Verfahren zum Nachweisen eines Zielnukleinsäuremoleküls in einer Probe, wobei das Verfahren Folgendes umfasst:
(i) Kontaktieren der Probe mit einer linearen Sonde, die an ihrem 5'- und 3'-Ende einen Zielbindungsbereich umfasst, der komplementär zu Sondenbindungsstellen in dem Zielnukleinsäuremolekül ist;
(ii) Kontaktieren der linearen Sonde mit einem inaktivierenden Oligonukleotid, das einen ersten und einen zweiten inaktivierenden Bereich umfasst, der komplementär zu den Zielbindungsbereichen an den Enden der linearen Sonde ist, wobei die zwei inaktivierenden Bereiche durch mindestens 5 Nukleotide voneinander getrennt sind und wobei das Kontaktieren vor, während oder nach dem Kontaktieren der Probe mit der linearen Sonde stattfindet;
(iii) Erlauben der zielspezifischen Bindungsbereiche der linearen Sonde, sich, zur unmittelbaren oder mittelbaren Ligation zueinander, nebeneinandergestellt mit dem Zielnukleinsäuremolekül zu hybridisieren;
(iv) unmittelbares oder mittelbares Ligieren des 5'- und des 3'-Endes der linearen Sonde, um die lineare Sonde zu zirkularisieren;
(v) nachdem das inaktivierende Oligonukleotid mit der linearen Sonde kontaktiert wurde, Kontaktieren der Probe, welche die zirkularisierte lineare Sonde enthält, mit einer strangverdrängenden Polymerase und Durchführen einer RCA-Reaktion unter Verwendung der zirkularisierten linearen Sonde als eine RCA-Matrize, um ein RCA-Produkt (RCP) zu erzeugen;
(vi) Nachweisen des RCP, um die Zielnukleinsäuresequenz nachzuweisen.

2. Verfahren nach Anspruch 1, wobei das Verfahren in einer Lösung durchgeführt wird.

3. Verfahren nach Anspruch 1 oder Anspruch 2, wobei das Zielnukleinsäuremolekül ein Zielanalyt ist oder aus einem Zielnukleinsäureanalyten erzeugt wird oder ein Reporter für einen Zielanalyten ist.

4. Verfahren nach einem der Ansprüche 1 bis 3, wobei die lineare Sonde mit dem inaktivierenden Oligonukleotid vorhybridisiert ist und das Zielnukleinsäuremolekül das inaktivierende Oligonukleotid aus linearen Sonden verdrängt, die sich mit dem Zielnukleinsäuremolekül hybridisieren.

5. Verfahren nach einem der Ansprüche 1 bis 3, wobei die linearen Sonden und das inaktivierende Oligonukleotid gleichzeitig oder in einer Beimischung mit der Probe kontaktiert werden, die das Zielnukleinsäuremolekül enthält.

6. Verfahren nach einem der Ansprüche 1 bis 3, wobei das inaktivierende Oligonukleotid, nachdem es der linearen Sonde erlaubt wurde, sich mit dem Zielnukleinsäuremolekül zu hybridisieren, oder nach dem Ligationsschritt (iv) derart mit der Probe kontaktiert wird, dass es sich mit beliebigen ungebundenen oder unligierten linearen Sonden in der Probe hybridisiert.

7. Verfahren nach einem der Ansprüche 1 bis 6, wobei die inaktivierenden Bereiche an dem 5'- und dem 3'-Ende des inaktivierenden Oligonukleotids liegen.

8. Verfahren nach einem der Ansprüche 1 bis 7, wobei das inaktivierende Oligonukleotid einen dritten inaktivierenden Bereich zwischen dem ersten und dem zweiten inaktivierenden Bereich umfasst, der komplementär zu einer inaktivierenden Oligonukleotidbindungsstelle in der linearen Sonde ist, wobei die inaktivierende Oligonukleotidbindungsstelle innen in der linearen Sonde zwischen dem 5'- und dem 3'-Ende davon liegt.

9. Verfahren nach einem der Ansprüche 1 bis 8, wobei die lineare Sonde eine Nachweissequenz umfasst, wobei die Nachweissequenz es der linearen Sonde oder einem Amplikon oder einer umgekehrten Komplementversion davon erlaubt, nachgewiesen zu werden.

10. Verfahren nach Anspruch 9, wobei die Nachweissequenz eine Bindungsstelle für ein Nachweisoligonukleotid ist oder eine Strichcodesequenz umfasst, gegebenenfalls wobei das Nachweisoligonukleotid mit einem Nachweisanteil verknüpft ist, bevorzugt wobei der Nachweisanteil ein Tropfen, ein fluoreszierendes oder kolorimetrisches Label, ein Farbstoff oder ein Enzymsubstrat ist.

11. Verfahren nach Anspruch 8 und einem der Ansprüche 9 bis 10, wobei sich die Nachweissequenz teilweise oder sämtlich mit der inaktivierenden Oligonukleotidbindungsstelle überlappt, die komplementär zu dem dritten inaktivierenden Bereich des inaktivierenden Oligonukleotids ist.

12. Verfahren nach einem der Ansprüche 1 bis 11, wobei das Verfahren multiplex durchgeführt wird, um zwei oder mehrere Zielnukleinsäuremoleküle in der Probe nachzuweisen, und wobei Schritt (i) Kontaktieren der Probe mit zwei oder mehreren linearen Sonden umfasst, die jeweils spezifisch für eine unterschiedliche Zielnukleinsäure sind, und ein unterschiedliches inaktivierendes Oligonukleotid für jede unterschiedliche lineare Sonde bereitgestellt wird.

13. Verfahren nach einem der Ansprüche 1 bis 12, wobei das Verfahren in als einzelnem Reaktionsbehälter durchgeführt wird.

14. Kit zum Nachweisen eines Zielnukleinsäuremoleküls in einer Probe, wobei das Kit Folgendes umfasst:
(i) eine lineare Sonde, die an ihrem 5'- und 3'-Ende einen Zielbindungsbereich umfasst, der komplementär zu Sondenbindungsstellen in dem Zielnukleinsäuremolekül ist;
(ii) ein inaktivierendes Oligonukleotid, das einen ersten und einen zweiten inaktivierenden Bereich umfasst, der komplementär zu den Zielbindungsbereichen an den Enden der linearen Sonde ist, wobei die zwei inaktivierenden Bereiche durch mindestens 5 Nukleotide voneinander getrennt sind; und
(iii) eine strangverdrängende Polymerase.

15. Kit nach Anspruch 14, wobei das Kit zwei oder mehrere unterschiedliche lineare Sonden, die jeweils spezifisch für ein unterschiedliches Zielmolekül sind, und zwei oder mehrere inaktivierende Oligonukleotide, die jeweils spezifisch für eine unterschiedliche lineare Sonde sind, umfasst.

16. Reagens, umfassend:
ein inaktivierendes Oligonukleotid zum Blockieren der Zielbindungsbereiche einer linearen Sonde; und
die lineare Sonde, die mit dem inaktivierenden Oligonukleotid hybridisiert ist, wobei das inaktivierende Oligonukleotid einen ersten und einen zweiten inaktivierenden Bereich umfasst, der komplementär zu den Zielbindungsbereichen an den Enden der linearen Sonde ist, wobei die zwei inaktivierenden Bereiche durch mindestens 5 Nukleotide voneinander getrennt sind und wobei das inaktivierende Oligonukleotid ferner einen dritten inaktivierenden Bereich umfasst, der zwischen dem ersten und dem zweiten inaktivierenden Bereich liegt und der komplementär zu einer inaktivierenden Oligonukleotidbindungsstelle in der linearen Sonde ist, die zwischen den Zielbindungsbereichen der linearen Sonde liegt, wobei die inaktivierende Oligonukleotidbindungsstelle durch Sequenzen, die nicht komplementär zu dem inaktivierenden Oligonukleotid sind, von den Zielbindungsbereichen in der linearen Sonde getrennt ist.

## Revendications

1. Procédé de détection d'une molécule d'acide nucléique cible dans un échantillon, ledit procédé comprenant :
(i) la mise en contact de l'échantillon avec une sonde cadenas, qui comprend à ses extrémités 5' et 3' des régions de liaison à la cible qui sont complémentaires de sites de liaison de sonde dans la molécule d'acide nucléique cible ;
(ii) la mise en contact de la sonde cadenas avec un oligonucléotide de silençage qui comprend des première et deuxième régions de silençage qui sont complémentaires des régions de liaison à la cible aux extrémités de la sonde cadenas, dans lequel les deux régions de silençage sont séparées l'une de l'autre par au moins 5 nucléotides, et dans lequel ladite mise en contact se produit avant, pendant ou après la mise en contact de l'échantillon avec la sonde cadenas ;
(iii) l'hybridation des régions de liaison spécifiques de la cible de la sonde cadenas à la molécule d'acide nucléique cible en juxtaposition pour une ligation, directement ou indirectement, l'une à l'autre ;
(iv) la ligation, directement ou indirectement, des extrémités 5' et 3' de la sonde cadenas pour circulariser la sonde cadenas ;
(v) après que l'oligonucléotide de silençage a été mis en contact avec la sonde cadenas, la mise en contact de l'échantillon contenant la sonde cadenas circularisée avec une polymérase à déplacement de brin et la réalisation d'une réaction d'amplification par cercle roulant, RCA, en utilisant la sonde cadenas circularisée comme matrice d'amplification par cercle roulant, RCA, pour générer un produit de RCA (RCP) ;
(vi) la détection du RCP pour détecter la séquence d'acide nucléique cible.

2. Procédé selon la revendication 1, dans lequel le procédé est réalisé en solution.

3. Procédé selon la revendication 1 ou la revendication 2, dans lequel la molécule d'acide nucléique cible est un analyte cible, ou est générée à partir d'un analyte d'acide nucléique cible ou est un rapporteur pour un analyte cible.

4. Procédé selon l'une quelconque des revendications 1 à 3, dans lequel la sonde cadenas est pré-hybridée à l'oligonucléotide de silençage, et la molécule d'acide nucléique cible déplace l'oligonucléotide de silençage des sondes cadenas qui s'hybrident à la molécule d'acide nucléique cible.

5. Procédé selon l'une quelconque des revendications 1 à 3, dans lequel les sondes cadenas et l'oligonucléotide de silençage sont mis en contact simultanément, ou en mélange, avec l'échantillon contenant la molécule d'acide nucléique cible.

6. Procédé selon l'une quelconque des revendications 1 à 3, dans lequel l'oligonucléotide de silençage est mis en contact avec l'échantillon après qu'il a été permis à la sonde cadenas de s'hybrider à la molécule d'acide nucléique cible, ou après l'étape de ligation (iv), de sorte qu'il s'hybride à toutes sondes cadenas non liées ou non liguées dans l'échantillon.

7. Procédé selon l'une quelconque des revendications 1 à 6, dans lequel les régions de silençage se situent aux extrémités 5' et 3' de l'oligonucléotide de silençage.

8. Procédé selon l'une quelconque des revendications 1 à 7, dans lequel l'oligonucléotide de silençage comprend une troisième région de silençage, entre les première et deuxième régions de silençage, qui est complémentaire d'un site de liaison d'oligonucléotide de silençage dans la sonde cadenas, dans lequel ledit site de liaison d'oligonucléotide de silençage se situe de manière interne dans la sonde cadenas, entre les extrémités 5' et 3' de celle-ci.

9. Procédé selon l'une quelconque des revendications 1 à 8, dans lequel la sonde cadenas comprend une séquence de détection, dans lequel la séquence de détection permet à la sonde cadenas, ou à un amplicon ou à une copie complémentaire inverse de celle-ci d'être détecté.

10. Procédé selon la revendication 9, dans lequel la séquence de détection est un site de liaison pour un oligonucléotide de détection, ou comprend une séquence code-barres, éventuellement dans lequel l'oligonucléotide de détection est lié à une entité de détection, de préférence dans lequel l'entité de détection est une bille, un marqueur fluorescent ou colorimétrique, un colorant, ou un substrat enzymatique.

11. Procédé selon la revendication 8 et l'une quelconque des revendications 9 à 10, dans lequel la séquence de détection chevauche partiellement ou totalement le site de liaison d'oligonucléotide de silençage qui est complémentaire de la troisième région de silençage de l'oligonucléotide de silençage.

12. Procédé selon l'une quelconque des revendications 1 à 11, dans lequel le procédé est réalisé en multiplex pour détecter deux ou plusieurs molécules d'acide nucléique cibles dans l'échantillon, et dans lequel l'étape (i) comprend la mise en contact de l'échantillon avec deux ou plusieurs sondes cadenas chacune spécifique pour un acide nucléique cible différent, et un oligonucléotide de silençage différent est fourni pour chaque sonde cadenas différente.

13. Procédé selon l'une quelconque des revendications 1 à 12, dans lequel le procédé est réalisé dans un seul récipient de réaction.

14. Kit pour détecter une molécule d'acide nucléique cible dans un échantillon, ledit kit comprenant :
(i) une sonde cadenas qui comprend à ses extrémités 5' et 3' des régions de liaison à la cible qui sont complémentaires de sites de liaison de sonde dans la molécule d'acide nucléique cible ;
(ii) un oligonucléotide de silençage qui comprend des première et deuxième régions de silençage qui sont complémentaires des régions de liaison à la cible aux extrémités de la sonde cadenas, dans lequel les deux régions de silençage sont séparées l'une de l'autre par au moins 5 nucléotides ; et
(iii) une polymérase à déplacement de brin.

15. Kit selon la revendication 14, dans lequel le kit comprend deux ou plusieurs sondes cadenas différentes, chacune spécifique pour une molécule cible différente, et deux ou plusieurs oligonucléotides de silençage chacun spécifique pour une sonde cadenas différente.

16. Réactif comprenant :
un oligonucléotide de silençage pour bloquer les régions de liaison à la cible d'une sonde cadenas ; et
la sonde cadenas hybridée à l'oligonucléotide de silençage, dans lequel ledit oligonucléotide de silençage comprenant des première et deuxième régions de silençage qui sont complémentaires des régions de liaison à la cible aux extrémités de la sonde cadenas, dans lequel les deux régions de silençage sont séparées l'une de l'autre par au moins 5 nucléotides, et dans lequel l'oligonucléotide de silençage comprend en outre une troisième région de silençage, qui se situe entre les première et deuxième régions de silençage et qui est complémentaire d'un site de liaison d'oligonucléotide de silençage dans la sonde cadenas qui se situe entre les régions de liaison à la cible de la sonde cadenas, dans lequel le site de liaison d'oligonucléotide de silençage est séparé des régions de liaison à la cible dans la sonde cadenas par des séquences qui ne sont pas complémentaires de l'oligonucléotide de silençage.
